# EUROPEAN PATENT SPECIFICATION

(11) **EP 4 348 668 B1**
(45) Date of publication and mention of the grant of the patent: **01.07.2026**
(21) Application number: 22732880.4
(22) Date of filing: 26.05.2022
(51) Int. Cl.: G16H 20/40, A61N 5/10, G06N 20/00, G06N 3/02

(54) **DISCREET PARAMETER AUTOMATED PLANNING**
DISKRETE AUTOMATISIERTE PARAMETERPLANUNG
PLANIFICATION AUTOMATISÉE DE PARAMÈTRES DISCRETS

(30) Priority: 02.06.2021 US 202163202235 P
(43) Date of publication of application: 10.04.2024
(73) Proprietor: Elekta, Inc., Atlanta GA 30346 (US)
(72) Inventor: STARBUCK, William Alvin, Atlanta, Georgia 30346 (US); LOPES, Rui, Atlanta, Georgia 30346 (US)
(74) Representative: Kilburn & Strode LLP
(86) International application number: PCT/US2022/072587
(87) International publication number: WO 2022/256782

(56) References cited:
- WO-A1-2020/256750
- US-A1- 2019 030 370
- US-A1- 2019 192 880
- US-A1- 2019 371 450
- US-A1- 2020 388 371
- US-A1- 2021 090 694
- US-A1- 2021 118 136
- US-A1- 2021 125 731

## Description

### TECHNICAL FIELD

Embodiments of the present disclosure pertain generally to treatment modality selection.

### BACKGROUND

Radiation therapy (or "radiotherapy") can be used to treat cancers or other ailments in mammalian (e.g., human and animal) tissue. One such radiotherapy technique involves irradiation with a Gamma Knife, whereby a patient is irradiated by a large number of low-intensity gamma ray beams that converge with high intensity and high precision at a target (e.g., a tumor). In another embodiment, radiotherapy is provided using a linear accelerator, whereby a tumor is irradiated by high-energy particles (e.g., electrons, protons, ions, high-energy photons, and the like). The placement and dose of the radiation beam must be accurately controlled to ensure the tumor receives the prescribed radiation, and the placement of the beam should be such as to minimize damage to the surrounding healthy tissue, often called the organ(s) at risk (OARs). Radiation is termed "prescribed" because a physician orders a predefined amount of radiation to the tumor and surrounding organs similar to a prescription for medicine. Generally, ionizing radiation in the form of a collimated beam is directed from an external radiation source toward a patient.

A specified or selectable beam energy can be used, such as for delivering a diagnostic energy level range or a therapeutic energy level range. Modulation of a radiation beam can be provided by one or more attenuators or collimators (e.g., a multi-leaf collimator (MLC)). The intensity and shape of the radiation beam can be adjusted by collimation to avoid damaging healthy tissue (e.g., OARs) adjacent to the targeted tissue by conforming the projected beam to a profile of the targeted tissue.

The treatment planning procedure may include using a three-dimensional (3D) image of the patient to identify a target region (e.g., the tumor) and to identify critical organs near the tumor. Creation of a treatment plan can be a time-consuming process where a planner tries to comply with various treatment objectives or constraints (e.g., dose volume histogram (DVH), overlap volume histogram (OVH)), taking into account their individual importance (e.g., weighting) in order to produce a treatment plan that is clinically acceptable. This task can be a time-consuming trial-and-error process that is complicated by the various OARs because as the number of OARs increases (e.g., up to thirteen for a head-and-neck treatment), so does the complexity of the process. OARs distant from a tumor may be easily spared from radiation, while OARs close to or overlapping a target tumor may be difficult to spare.

Traditionally, for each patient, the initial treatment plan can be generated in an "offline" manner. The treatment plan can be developed well before radiation therapy is delivered, such as using one or more medical imaging techniques. Imaging information can include, for example, images from X-rays, computed tomography (CT), nuclear magnetic resonance (MR), positron emission tomography (PET), single-photon emission computed tomography (SPECT), or ultrasound. A health care provider, such as a physician, may use 3D imaging information indicative of the patient anatomy to identify one or more target tumors along with the OARs near the tumor(s). The health care provider can delineate the target tumor that is to receive a prescribed radiation dose using a manual technique, and the health care provider can similarly delineate nearby tissue, such as organs, at risk of damage from the radiation treatment. Alternatively, or additionally, an automated tool (e.g., ABAS provided by Elekta AB, Sweden) can be used to assist in identifying or delineating the target tumor and organs at risk. A radiation therapy treatment plan ("treatment plan") can then be created using an optimization technique based on clinical and dosimetric objectives and constraints (e.g., the maximum, minimum, and fraction of dose of radiation to a fraction of the tumor volume ("95% of target shall receive no less than 100% of prescribed dose"), and like measures for the critical organs). The optimized plan is comprised of numerical parameters that specify the direction, cross-sectional shape, and intensity of each radiation beam.

The treatment plan can then be later executed by positioning the patient in the treatment machine and delivering the prescribed radiation therapy directed by the optimized plan parameters. The radiation therapy treatment plan can include dose "fractioning," whereby a sequence of radiation treatments is provided over a predetermined period of time (e.g., 30-45 daily fractions), with each treatment including a specified fraction of a total prescribed dose. However, during treatment, the position of the patient and the position of the target tumor in relation to the treatment machine (e.g., linear accelerator - "linac") is very important in order to ensure the target tumor and not healthy tissue is irradiated.

US2019371450A1 describes decision support in a medical therapy, wherein machine learning provides a machine-learned generator for generating a prediction of outcome for therapy personalized to a patient. Learning may be provided by using multi-task learning where one of the tasks (e.g., segmentation, non-image data, and/or feature extraction) is unsupervised and/or draws on a greater number of training samples than available for outcome prediction alone.

US2021125731A1 describes a system and method for analyzing a data store of de-identified patient data to generate one or more dynamic user interfaces usable to predict an expected response of a particular patient population or cohort when provided with a certain treatment.

### BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example but not by way of limitation, various embodiments discussed in the present document.
FIG. 1 illustrates an example radiotherapy system including a treatment modality selection device, according to some embodiments of the present disclosure.
FIG. 2A illustrates an example radiation therapy system that can include radiation therapy output configured to provide a therapy beam, according to some embodiments of the present disclosure.
FIG. 2B illustrates an example system including a combined radiation therapy system and an imaging system, such as a cone beam computed tomography (CBCT) imaging system, according to some embodiments of the present disclosure.
FIG. 3 illustrates a partially cut-away view of an example system including a combined radiation therapy system and an imaging system, such as a nuclear MR imaging (MRI) system, according to some embodiments of the present disclosure.
FIGS. 4A and 4B depict the differences between an example MRI image and a corresponding CT image, respectively, according to some embodiments of the present disclosure.
FIG. 5 illustrates an example collimator configuration for shaping, directing, or modulating an intensity of a radiation therapy beam, according to some embodiments of the present disclosure.
FIG. 6 illustrates an example Gamma Knife radiation therapy system, according to some embodiments of the present disclosure.
FIG. 7 illustrates an example flow diagram for deep learning, according to some embodiments of the present disclosure.
FIG. 8 illustrates an example of a treatment modality selection system, according to some embodiments of the present disclosure.

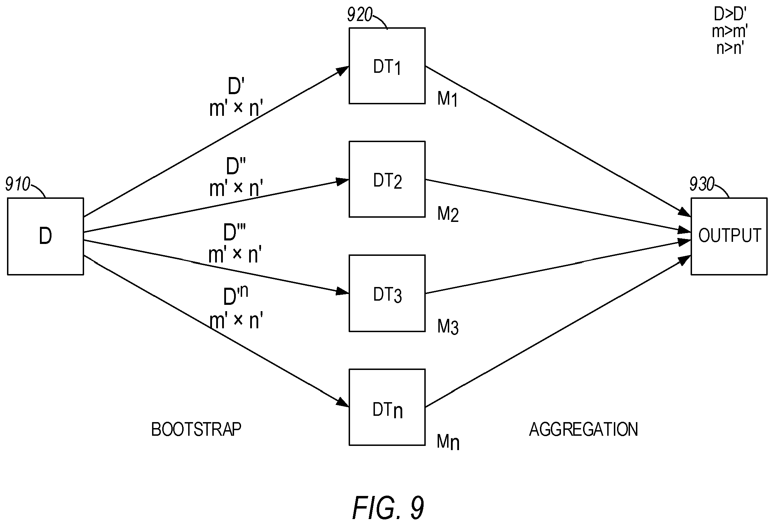

FIG. 9 illustrates example multi-parametric input data processing, according to some embodiments of the present disclosure.
FIG. 10 illustrates an example output of the treatment modality selection system, according to some embodiments of the present disclosure.
FIG. 11 illustrates an example data flow for training and use of a machine learning model to generate modalities for treating disease, according to some embodiments of the present disclosure.
FIG. 12 illustrates a method for selecting a treatment modality, according to some embodiments of the present disclosure.
FIG. 13 illustrates an example block diagram of a machine on which one or more of the methods as discussed herein can be implemented.

### DETAILED DESCRIPTION

In the following detailed description, reference is made to the accompanying drawings which form a part hereof, and which is shown by way of illustration-specific embodiments in which the present disclosure may be practiced. These embodiments, which are also referred to herein as "examples," are described in sufficient detail to enable those skilled in the art to practice the disclosure, and it is to be understood that the embodiments may be combined, or that other embodiments may be utilized and that structural, logical and electrical changes may be made without departing from the scope of the present

Disease markup and annotation by the physician has inherent variability and bias based on education, residence, practice, experience and exposure. Specifically, the course of treatment for a given disease is usually guided by who the physician is and what experience the physician has with the given disease. The physician may not be up-to-date with the latest standards for treatment and may prescribe a treatment modality for the disease that no longer comports with the current standards. Given the large variability and many types of treatment modalities that can be selected to treat a given disease, patients are put at risk by visiting a physician who is not as experienced and may not be as informed as other physicians. In some cases, a physician may not be aware of all the medical history associated with a patient before prescribing a particular course of treatment or treatment modality. This can severely impact the success or survival rate of treating a given disease. Template-based planning can help reduce variability but may not encompass an extent of personalization or nuance of disease metrics in both the planning images and the patient's electronic medical record (EMR). Also, reviewing all of the medical history for a given patient to come up with a course of treatment (e.g., a treatment modality) is very time consuming, expensive, and can still result in some information being missed.

The disclosed embodiments address these challenges by providing a system that recommends or selects a course of treatment for a given disease based on multiple input parameters associated with a patient. The disclosed embodiments receive multi-parametric input data representing data associated with a patient and receive an indication of a disease associated with the patient. The disclosed embodiments process the multi-parametric input data to generate one or more metrics corresponding to a plurality of different modalities for treating the disease associated with the patient. The disclosed embodiments select, based on the one or more metrics, a given modality from the plurality of different modalities to treat the disease associated with the patient and configure parameters of the given modality based on a portion of the multi-parametric input data. As an example, a machine learning technique can be trained to generate a set of treatment modalities for treating the disease based on the multi-parametric input data. Specifically, the machine learning technique can be trained to establish a relationship between a plurality of characteristics of pre-treatment planning associated with known patients associated with the disease, the modality of the plurality of different modalities used to treat the disease for each of the known patients, and a treatment result of each of the known patients. The machine learning technique can output different weights and metrics of different treatment modalities and a physician or user can use these weights and metrics to select a course of treatment. In some cases, the machine learning technique automatically selects a top ranked course of treatment based on the weights and metrics and recommends the top ranked course of treatment be used to treat the disease.

In this way, an automated system is provided that recommends a treatment modality in a fast and efficient manner. This saves computational resources and the time and expense usually incurred by a physician in prescribing a course of treatment. By automating the process used to select a treatment modality for treating disease given a set of patient data or characteristics, the course of treatment selected for treating the disease can be standardized across a pool of patients in a given region or population. This reduces the amount of variability and discrepancies in results obtained by treating patients associated with a given disease. Also, by automating the manner in which a modality of treatment is selected, the latest standards, rules and regulations set by governing or professional bodies can be considered in generating the metrics that are used to select the treatment modality. In some cases, the automated system can detect that a course of treatment selected to treat the disease differs from that which is standardized or recommended by the automated system. In such circumstances, the automated system can be used for quality control and to generate a prompt, such as if the result obtained by the selected course of treatment has a treatment score that exceeds a specified threshold amount from a score associated with an expected result of the recommended course of treatment. This can be used to flag certain physicians and the protocols those physicians use to improve the overall treatment provided by a given facility.

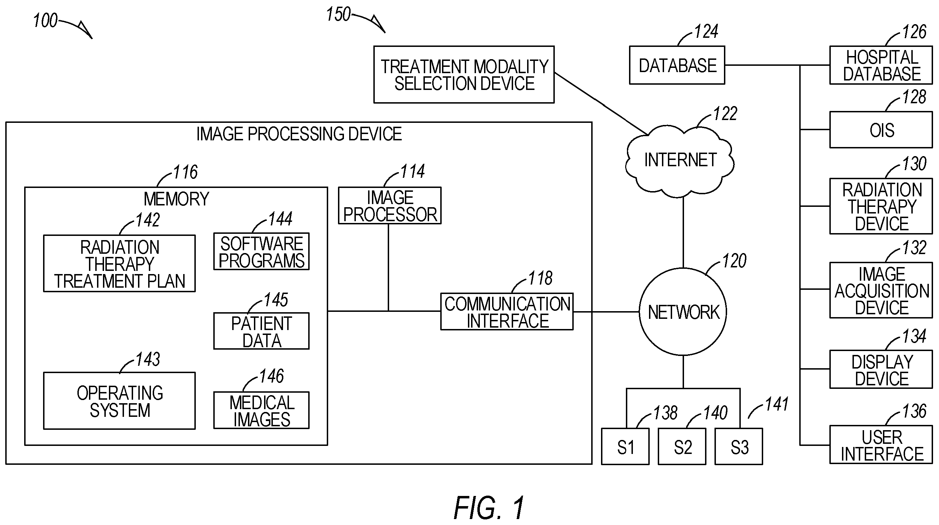

FIG. **1** illustrates an example radiotherapy system 100 for providing radiation therapy to a patient, according to some embodiments. The radiotherapy system 100 includes an image processing device 112. The image processing device 112 may be connected to a network 120. The network 120 may be connected to the Internet 122. The network 120 can connect the image processing device 112 with one or more of a database 124, a hospital database 126, an oncology information system (OIS) 128, a radiation therapy device 130, an image acquisition device 132, a display device 134, and a user interface 136. The image processing device 112 can be configured to generate radiation therapy treatment plans 142 to be used by the radiation therapy device 130. The radiotherapy system 100 includes a treatment modality selection device 150. The treatment modality selection device 150 can connect to the Internet 122 to communicate with any of the components shown in FIG. 1, such as the image processing device 112, the database 124, the user interface 136, the display device 134, and so forth. While the disclosed embodiments are discussed in connection with radiotherapy system 100 (e.g., a radiotherapy treatment modality), the techniques described herein for selecting a treatment modality apply similarly to any other treatment modality, such as a surgical modality, a chemotherapy modality, immunotherapy modality, hormone therapy modality, stem cell transplant modality, precision medicine modality, or any combination thereof.

The image processing device 112 may include a memory device 116, a processor 114, and a communication interface 118. The memory device 116 may store computer-executable instructions, such as an operating system 143, radiation therapy treatment plans 142 (e.g., original treatment plans, adapted treatment plans and the like), software programs 144 (e.g., artificial intelligence, deep learning, neural networks, radiotherapy treatment plan software), and any other computer-executable instructions to be executed by the processor 114.

In one embodiment, the software programs 144 may convert medical images of one format (e.g., MRI) to another format (e.g., CT) by producing synthetic images, such as pseudo-CT images. For instance, the software programs 144 may include image processing programs to train a predictive model for converting a medical image 146 in one modality (e.g., an MRI image) into a synthetic image of a different modality (e.g., a pseudo CT image); alternatively, the trained predictive model may convert a CT image into an MRI image. In another embodiment, the software programs 144 may register the patient image (e.g., a CT image or an MR image) with that patient's dose distribution (also represented as an image) so that corresponding image voxels and dose voxels are associated appropriately by the network. In yet another embodiment, the software programs 144 may substitute functions of the patient images or processed versions of the images that emphasize some aspect of the image information. Such functions might emphasize edges or differences in voxel textures, or any other structural aspect useful to neural network learning. In another embodiment, the software programs 144 may substitute functions of the dose distribution that emphasize some aspect of the dose information. Such functions might emphasize steep gradients around the target or any other structural aspect useful to neural network learning. The memory device 116 may store data, including medical images 146, patient data 145, and other data required to create and implement a radiation therapy treatment plan 142.

In addition to the memory device 116 storing the software programs 144, it is contemplated that software programs 144 may be stored on a removable computer medium, such as a hard drive, a computer disk, a CD-ROM, a DVD, a HD, a Blu-Ray DVD, USB flash drive, a SD card, a memory stick, or any other suitable medium; and the software programs 144 when downloaded to image processing device 112 may be executed by image processor 114.

The processor 114 may be communicatively coupled to the memory device 116, and the processor 114 may be configured to execute computer-executable instructions stored thereon. The processor 114 may send or receive medical images 146 to memory device 116. For example, the processor 114 may receive medical images 146 from the image acquisition device 132 via the communication interface 118 and network 120 to be stored in memory device 116. The processor 114 may also send medical images 146 stored in memory device 116 via the communication interface 118 to the network 120 be either stored in database 124 or the hospital database 126.

Further, the processor 114 may utilize software programs 144 (e.g., a treatment planning software) along with the medical images 146 and patient data 145 to create the radiation therapy treatment plan 142. Medical images 146 may include information such as imaging data associated with a patient anatomical region, organ, or volume of interest segmentation data. Patient data 145 may include information such as (1) functional organ modeling data (e.g., serial versus parallel organs, appropriate dose response models, etc.); (2) radiation dosage data (e.g., DVH information); or (3) other clinical information about the patient and course of treatment (e.g., other surgeries, chemotherapy, previous radiotherapy, etc.).

In addition, the processor 114 may utilize software programs to generate intermediate data such as updated parameters to be used, for example, by a machine learning model, such as a neural network model; or generate intermediate 2D or 3D images, which may then subsequently be stored in memory device 116. The processor 114 may subsequently transmit the executable radiation therapy treatment plan 142 via the communication interface 118 to the network 120 to the radiation therapy device 130, where the radiation therapy plan will be used to treat a patient with radiation. In addition, the processor 114 may execute software programs 144 to implement functions such as image conversion, image segmentation, deep learning, neural networks, and artificial intelligence. For instance, the processor 114 may execute software programs 144 that train or contour a medical image; such software programs144 when executed may train a boundary detector or utilize a shape dictionary.

The processor 114 may be a processing device, include one or more general-purpose processing devices such as a microprocessor, a central processing unit (CPU), a graphics processing unit (GPU), an accelerated processing unit (APU), or the like. More particularly, the processor 114 may be a complex instruction set computing (CISC) microprocessor, a reduced instruction set computing (RISC) microprocessor, a very long instruction Word (VLIW) microprocessor, a processor implementing other instruction sets, or processors implementing a combination of instruction sets. The processor 114 may also be implemented by one or more special-purpose processing devices such as an application-specific integrated circuit (ASIC), a field programmable gate array (FPGA), a digital signal processor (DSP), a System on a Chip (SoC), or the like. As would be appreciated by those skilled in the art, in some embodiments, the processor 114 may be a special-purpose processor, rather than a general-purpose processor. The processor 114 may include one or more known processing devices, such as a microprocessor from the Pentium^{™}, Core^{™}, Xeon^{™}, or Itanium^{®} family manufactured by Intel^{™}, the Turion^{™}, Athlon^{™}, Sempron^{™}, Opteron^{™}, FX^{™}, Phenom^{™} family manufactured by AMD^{™}, or any of various processors manufactured by Sun Microsystems. The processor 114 may also include graphical processing units such as a GPU from the GeForce^{®}, Quadro^{®}, Tesla^{®} family manufactured by Nvidia^{™}, GMA, Iris^{™} family manufactured by Intel^{™}, or the Radeon^{™} family manufactured by AMD^{™}, The processor 114 may also include accelerated processing units such as the Xeon Phi^{™} family manufactured by Intel^{™}. The disclosed embodiments are not limited to any type of processor(s) otherwise configured to meet the computing demands of identifying, analyzing, maintaining, generating, and/or providing large amounts of data or manipulating such data to perform the methods disclosed herein. In addition, the term "processor" may include more than one processor (for example, a multi-core design or a plurality of processors each having a multi-core design). The processor 114 can execute sequences of computer program instructions, stored in memory device 116, to perform various operations, processes, methods that will be explained in greater detail below.

The memory device 116 can store medical images 146. In some embodiments, the medical images 146 may include one or more MRI images (e.g., 2D MRI, 3D MRI, 2D streaming MRI, four-dimensional (4D) MRI, 4D volumetric MRI, 4D cine MRI, etc.), functional MRI images (e.g., fMRI, DCE-MRI, diffusion MRI), CT images (e.g., 2D CT, cone beam CT, 3D CT, 4D CT), ultrasound images (e.g., 2D ultrasound, 3D ultrasound, 4D ultrasound), one or more projection images representing views of an anatomy depicted in the MRI, synthetic CT (pseudo-CT), and/or CT images at different angles of a gantry relative to a patient axis, PET images, X-ray images, fluoroscopic images, radiotherapy portal images, SPECT images, computer-generated synthetic images (e.g., pseudo-CT images), aperture images, graphical aperture image representations of MLC leaf positions at different gantry angles, and the like. Further, the medical images 146 may also include medical image data, for instance, training images, ground truth images, contoured images, and dose images. In an embodiment, the medical images 146 may be received from the image acquisition device 132. Accordingly, image acquisition device 132 may include an MRI imaging device, a CT imaging device, a PET imaging device, an ultrasound imaging device, a fluoroscopic device, a SPECT imaging device, an integrated linac and MRI imaging device, or other medical imaging devices for obtaining the medical images of the patient. The medical images 146 may be received and stored in any type of data or any type of format that the image processing device 112 may use to perform operations consistent with the disclosed embodiments.

The memory device 116 may be a non-transitory computer-readable medium, such as a read-only memory (ROM), a phase-change random access memory (PRAM), a static random access memory (SRAM), a flash memory, a random access memory (RAM), a dynamic random access memory (DRAM) such as synchronous DRAM (SDRAM), an electrically erasable programmable read-only memory (EEPROM), a static memory (e.g., flash memory, flash disk, static random access memory) as well as other types of random access memories, a cache, a register, a CD-ROM, a DVD or other optical storage, a cassette tape, other magnetic storage device, or any other non-transitory medium that may be used to store information including image, data, or computer executable instructions (e.g., stored in any format) capable of being accessed by the processor 114, or any other type of computer device. The computer program instructions can be accessed by the processor 114, read from the ROM, or any other suitable memory location, and loaded into the RAM for execution by the processor 114. For example, the memory device 116 may store one or more software applications. Software applications stored in the memory device 116 may include, for example, an operating system 143 for common computer systems as well as for software-controlled devices. Further, the memory device 116 may store an entire software application, or only a part of a software application, that is executable by the processor 114. For example, the memory device 116 may store one or more radiation therapy treatment plans 142.

The image processing device 112 can communicate with the network 120 via the communication interface 118, which can be communicatively coupled to the processor 114 and the memory device 116. The communication interface 118 may provide communication connections between the image processing device 112 and radiotherapy system 100 components (e.g., permitting the exchange of data with external devices). For instance, the communication interface 118 may, in some embodiments, have appropriate interfacing circuitry to connect to the user interface 136, which may be a hardware keyboard, a keypad, or a touch screen through which a user may input information into radiotherapy system 100.

Communication interface 118 may include, for example, a network adaptor, a cable connector, a serial connector, a USB connector, a parallel connector, a high-speed data transmission adaptor (e.g., such as fiber, USB 3.0, thunderbolt, and the like), a wireless network adaptor (e.g., such as a WiFi adaptor), a telecommunication adaptor (e.g., 3G. 4G/LTE and the like), and the like. Communication interface 118 may include one or more digital and/or analog communication devices that permit image processing device 112 to communicate with other machines and devices, such as remotely located components, via the network 120.

The network 120 may provide the functionality of a local area network (LAN), a wireless network, a cloud computing environment (e.g., software as a service, platform as a service, infrastructure as a service, etc.), a client-server, a wide area network (WAN), and the like. For example, network 120 may be a LAN or a WAN that may include other systems S1 (138), S2 (140), and S3 (141). Systems S1, S2, and S3 may be identical to image processing device 112 or may be different systems. In some embodiments, one or more systems in network 120 may form a distributed computing/simulation environment that collaboratively performs the embodiments described herein. In some embodiments, one or more systems S1, S2, and S3 may include a CT scanner that obtains CT images (e.g., medical images 146). In addition, network 120 may be connected to Internet 122 to communicate with servers and clients that reside remotely on the internet.

Therefore, network 120 can allow data transmission between the image processing device 112 and a number of various other systems and devices, such as the OIS 128, the radiation therapy device 130, and the image acquisition device 132. Further, data generated by the OIS 128 and/or the image acquisition device 132 may be stored in the memory device 116, the database 124, and/or the hospital database 126. The data may be transmitted/received via network 120, through communication interface 118 in order to be accessed by the processor 114, as required.

The image processing device 112 may communicate with database 124 through network 120 to send/receive a plurality of various types of data stored on database 124. For example, database 124 may include machine data (control points) that includes information associated with a radiation therapy device 130, image acquisition device 132, or other machines relevant to radiotherapy. Machine data information may include control points, such as radiation beam size, arc placement, beam on and off time duration, machine parameters, segments, MLC configuration, gantry speed, MRI pulse sequence, and the like. Database 124 may be a storage device and may be equipped with appropriate database administration software programs. One skilled in the art would appreciate that database 124 may include a plurality of devices located either in a central or a distributed manner.

In some embodiments, database 124 may include a processor-readable storage medium (not shown). While the processor-readable storage medium in an embodiment may be a single medium, the term "processor-readable storage medium" should be taken to include a single medium or multiple media (e.g., a centralized or distributed database, and/or associated caches and servers) that store the one or more sets of computer-executable instructions or data. The term "processor-readable storage medium" shall also be taken to include any medium that is capable of storing or encoding a set of instructions for execution by a processor and that cause the processor to perform any one or more of the methodologies of the present disclosure. The term "processor-readable storage medium" shall accordingly be taken to include, but not be limited to, solid-state memories and optical and magnetic media. For example, the processor-readable storage medium can be one or more volatile, non-transitory, or non-volatile tangible computer-readable media.

Image processor 114 may communicate with database 124 to read images into memory device 116 or store images from memory device 116 to database 124. For example, the database 124 may be configured to store a plurality of images (e.g., 3D MRI, 4D MRI, 2D MRI slice images, CT images, 2D Fluoroscopy images, X-ray images, raw data from MR scans or CT scans, Digital Imaging and Communications in Medicine (DIMCOM) data, projection images, graphical aperture images, etc.) that the database 124 received from image acquisition device 132. Database 124 may store data to be used by the image processor 114 when executing software program 144 or when creating radiation therapy treatment plans 142. Database 124 may store the data produced by the trained machine learning mode, such as a neural network including the network parameters constituting the model learned by the network and the resulting estimated data. As referred to herein, "estimate" or "estimated" can be used interchangeably with predict or predicted and should be understood to have the same meaning. The image processing device 112 may receive the imaging data, such as a medical image 146 (e.g., 2D MRI slice images, CT images, 2D Fluoroscopy images, X-ray images, 3DMRI images, 4D MRI images, projection images, graphical aperture images, image contours, etc.) from the database 124, the radiation therapy device 130 (e.g., an MR-linac), and/or the image acquisition device 132 to generate a treatment plan 142.

In an embodiment, the radiotherapy system 100 can include an image acquisition device 132 that can acquire medical images (e.g., MRI images, 3D MRI, 2D streaming MRI, 4D volumetric MRI, CT images, cone-Beam CT, PET images, functional MRI images (e.g., fMRI, DCE-MRI and diffusion MRI), X-ray images, fluoroscopic image, ultrasound images, radiotherapy portal images, SPECT images, and the like) of the patient. Image acquisition device 132 may, for example, be an MRI imaging device, a CT imaging device, a PET imaging device, an ultrasound device, a fluoroscopic device, a SPECT imaging device, or any other suitable medical imaging device for obtaining one or more medical images of the patient. Images acquired by the image acquisition device 132 can be stored within database 124 as either imaging data and/or test data. By way of example, the images acquired by the image acquisition device 132 can be also stored by the image processing device 112, as medical image 146 in memory device 116.

In an embodiment, for example, the image acquisition device 132 may be integrated with the radiation therapy device 130 as a single apparatus (e.g., an MR-linac). Such an MR-linac can be used, for example, to determine a location of a target organ or a target tumor in the patient, so as to direct radiation therapy accurately according to the radiation therapy treatment plan 142 to a predetermined target.

The image acquisition device 132 can be configured to acquire one or more images of the patient's anatomy for a region of interest (e.g., a target organ, a target tumor, or both). Each image, typically a 2D image or slice, can include one or more parameters (e.g., a 2D slice thickness, an orientation, and a location, etc.). In an embodiment, the image acquisition device 132 can acquire a 2D slice in any orientation. For example, an orientation of the 2D slice can include a sagittal orientation, a coronal orientation, or an axial orientation. The processor 114 can adjust one or more parameters, such as the thickness and/or orientation of the 2D slice, to include the target organ and/or target tumor. In an embodiment, 2D slices can be determined from information such as a 3D MRI volume. Such 2D slices can be acquired by the image acquisition device 132 in "real-time" while a patient is undergoing radiation therapy treatment, for example, when using the radiation therapy device 130, with "real-time" meaning acquiring the data in at least milliseconds or less.

The image processing device 112 may generate and store radiation therapy treatment plans 142 for one or more patients. The radiation therapy treatment plans 142 may provide information about a particular radiation dose to be applied to each patient. The radiation therapy treatment plans 142 may also include other radiotherapy information, such as control points including beam angles, gantry angles, beam intensity, dose-histogram-volume information, the number of radiation beams to be used during therapy, the dose per beam, and the like.

The image processor 114 may generate the radiation therapy treatment plan 142 by using software programs 144 such as treatment planning software (such as Monaco^{®}, manufactured by Elekta AB of Stockholm, Sweden). In order to generate the radiation therapy treatment plans 142, the image processor 114 may communicate with the image acquisition device 132 (e.g., a CT device, an MRI device, a PET device, an X-ray device, an ultrasound device, etc.) to access images of the patient and to delineate a target, such as a tumor, to generate contours of the images. In some embodiments, the delineation of one or more OARs, such as healthy tissue surrounding the tumor or in close proximity to the tumor, may be required. Therefore, segmentation of the OAR may be performed when the OAR is close to the target tumor. In addition, if the target tumor is close to the OAR (e.g., prostate in near proximity to the bladder and rectum), then by segmenting the OAR from the tumor, the radiotherapy system 100 may study the dose distribution not only in the target but also in the OAR.

In order to delineate a target organ or a target tumor from the OAR, medical images, such as MRI images, CT images, PET images, fMRI images, X-ray images, ultrasound images, radiotherapy portal images, SPECT images, and the like, of the patient undergoing radiotherapy may be obtained non-invasively by the image acquisition device 132 to reveal the internal structure of a body part. Based on the information from the medical images, a 3D structure of the relevant anatomical portion may be obtained and used to generate a contour of the image. Contours of the image can include data overlaid on top of the image that delineates one or more structures of the anatomy. In some cases, the contours can be files associated with respective images that specify the coordinates or 2D or 3D locations of various structures of the anatomy depicted in the images.

In addition, during a treatment planning process, many parameters may be taken into consideration to achieve a balance between efficient treatment of the target tumor (e.g., such that the target tumor receives enough radiation dose for an effective therapy) and low irradiation of the OAR(s) (e.g., the OAR(s) receives as low a radiation dose as possible). Other parameters that may be considered include the location of the target organ and the target tumor, the location of the OAR, and the movement of the target in relation to the OAR. For example, the 3D structure may be obtained by contouring the target or contouring the OAR within each 2D layer or slice of an MRI or CT image and combining the contour of each 2D layer or slice. The contour may be generated manually (e.g., by a physician, dosimetrist, or health care worker using a program such as MONACO^{™} manufactured by Elekta AB of Stockholm, Sweden) or automatically (e.g., using a program such as the Atlas-based auto-segmentation software, ABAS^{™}, manufactured by Elekta AB of Stockholm, Sweden). In certain embodiments, the 3D structure of a target tumor or an OAR may be generated automatically by the treatment planning software.

After the target tumor and the OAR(s) have been located and delineated, a dosimetrist, physician, or healthcare worker may determine a dose of radiation to be applied to the target tumor, as well as any maximum amounts of dose that may be received by the OAR proximate to the tumor (e.g., left and right parotid, optic nerves, eyes, lens, inner ears, spinal cord, brain stem, and the like). After the radiation dose is determined for each anatomical structure (e.g., target tumor, OAR), a process known as inverse planning may be performed to determine one or more treatment plan parameters that would achieve the desired radiation dose distribution. Examples of treatment plan parameters include volume delineation parameters (e.g., which define target volumes, contour sensitive structures, etc.), margins around the target tumor and OARs, beam angle selection, collimator settings, and beam-on times. During the inverse-planning process, the physician may define dose constraint parameters that set bounds on how much radiation an OAR may receive (e.g., defining full dose to the tumor target and zero dose to any OAR; defining 95% of dose to the target tumor; defining that the spinal cord, brain stem, and optic structures receive ≤ 45Gy, ≤ 55Gy and < 54Gy, respectively). The result of inverse planning may constitute a radiation therapy treatment plan 142 that may be stored in memory device 116 or database 124. Some of these treatment parameters may be correlated. For example, tuning one parameter (e.g., weights for different objectives, such as increasing the dose to the target tumor) in an attempt to change the treatment plan may affect at least one other parameter, which in turn may result in the development of a different treatment plan. Thus, the image processing device 112 can generate a tailored radiation therapy treatment plan 142 having these parameters in order for the radiation therapy device 130 to provide radiotherapy treatment to the patient.

In addition, the radiotherapy system 100 may include a display device 134 and a user interface 136. The display device 134 may include one or more display screens that display medical images, interface information, treatment planning parameters (e.g., projection images, graphical aperture images, contours, dosages, beam angles, etc.) treatment plans, a target, localizing a target and/or tracking a target, or any related information to the user. The user interface 136 may be a keyboard, a keypad, a touch screen or any type of device that a user may use to input information to radiotherapy system 100. Alternatively, the display device 134 and the user interface 136 may be integrated into a device such as a tablet computer (e.g., Apple iPad^{®}, Lenovo ThinkPad^{®}, Samsung Galaxy ^{®}, etc.).

Furthermore, any and all components of the radiotherapy system 100 may be implemented as a virtual machine (e.g., VMWare, Hyper-V, and the like). For instance, a virtual machine can be software that functions as hardware. Therefore, a virtual machine can include at least one or more virtual processors, one or more virtual memories, and one or more virtual communication interfaces that together function as hardware. For example, the image processing device 112, the OIS 128, and the image acquisition device 132 could be implemented as a virtual machine. Given the processing power, memory, and computational capability available, the entire radiotherapy system 100 could be implemented as a virtual machine.

The treatment modality selection device 150 may include similar components as the image processing device 112 with similar functionality. In some cases, the treatment modality selection device 150 is integrated as part of the image processing device 112. The treatment modality selection device 150 is configured to receive or access multi-parametric input data representing data associated with a patient. For example, the treatment modality selection device 150 accesses, as part of the multi-parametric input data, EMR information stored in the database 124 and imaging information stored in the medical images 146. In some cases, the treatment modality selection device 150 and/or the database 124 can also access a questionnaire filled out by a given patient to determine additional, more recent information about the patient than what is already stored in the database 124. The treatment modality selection device 150 and/or the database 124 can detect differences between data input in the questionnaire and the data stored in the database 124. The treatment modality selection device 150 and/or the database 124 can update the data stored in the database 124 for the patient with the more recent data input in the questionnaire. For example, the database 124 may lack an indication that the patient has recently had surgery to remove a kidney. The questionnaire may have a field requesting input from the patient about recent surgeries. The treatment modality selection device 150 and/or the database 124 can detect that the field has been populated with an indication that surgery was performed recently (in the past week) to remove a kidney. In response, the treatment modality selection device 150 and/or the database 124 automatically updates the corresponding EMR information stored in the database 124 to indicate that the patient has only one kidney.

The multi-parametric input **data** can include any combination of one or more image features, one or more features derived from **an image,** clinical **data, and EMR** information associated with the patient. The one or more image features can include a size, volume, or intensity of a region of interest, such as a prostate. The one or more features derived from an image can include radiomics features including texture and gradients of the one or more image features. The clinical data can include staging, genomics, and/or one or more tumor-specific features, such as: a Gleason score, a prostate-specific antigen (PSA) score, previous surgical intervention data, previous radiation treatments information, previous systemic therapies information, proximity to organs at risk data, obstructive symptoms information, presence of specific heritable pathogenic variants information, laterality information, morphology information, ethnicity information, gene panels information, genetic mutations information, ultrasound data, endoscopy data, physical examination results, urine test information, blood test information, biopsy data, Human papillomavirus infection (HPV) infection, gender, age, Epstein-Barr infection information, clear surgical margins data, Eastern Cooperative Oncology Group (ECOG) status information, radiosensitising conditions information, collagen vascular disease information, Non-invasive ductal carcinoma in situ information, body mass index (BMI) information, level of T-Cells in a body information, pregnancy status information, suitability of surgery information, and positron emission tomography (PET) staging information.

The multi-parametric input data can also include one or more outcome metrics, such as: toxicities, disease-free survival information for previous patients with the disease, or reimbursement information for a plurality of different treatment modalities. For example, the multi-parametric input data can include a table or list of diseases along with their respective survival rates for different treatment modalities. As referred to herein, a "modality" or "treatment modality" is defined as an intervention technique for treating a disease, such as performing surgery, performing radiotherapy, performing chemotherapy, performing immunotherapy, performing targeted therapy, performing hormone therapy, performing a stem cell transplant, performing precision medicine, any combination of performing surgery, performing radiotherapy, performing chemotherapy, performing immunotherapy, performing targeted therapy, performing hormone therapy, performing a stem cell transplant, performing precision medicine, and preventing performance of any treatment. The reimbursement information represents the amount of resources or money paid to the provider or physician for performing a given treatment modality. The multi-parametric input data can include for each disease and each treatment modality information representing how much radiation or toxicity resulted from implementing the given treatment modality and the survival information associated with the given treatment modality. As referred to herein, "treating a disease" can mean providing treatment to help lessen the symptoms and adverse effects of a disease, such as to reduce a size of a tumor or eliminate cancer cells. Disease treatment can involve one or multiple rounds of one or combination of different treatment modalities (e.g., therapy, surgery, medicine, and so forth).

The treatment modality selection device 150 can receive an indication of a disease associated with the patient. For example, the treatment modality selection device 150 can automatically process the multi-parametric input data to derive or determine a most likely disease (e.g., type of cancer, region of tumor cells, type of genetic disease, type of physiological disease, such as diabetes, multiple sclerosis, Crohn's & Colitis, lupus, rheumatoid arthritis, celiac disease, scleroderma, liver disease, cancer, heart disease, and so forth) associated with the patient. In another embodiment, the treatment modality selection device 150 can present a user interface to a provider or physician that allows the provider or physician to input the disease (e.g., type of cancer, region of tumor cells, type of genetic disease, type of physiological disease, such as diabetes, multiple sclerosis, Crohn's & Colitis, lupus, rheumatoid arthritis, celiac disease, scleroderma, liver disease, cancer, heart disease, and so forth) associated with the patient and for which the multi-parametric input data was received. In some cases, the user interface provides an abbreviated list of possible diseases (e.g., type of cancer and region of tumor cells) automatically selected by the treatment modality selection device 150 based on the multi-parametric input data. The treatment modality selection device 150 then receives a selection from the provider or physician from the abbreviated list of possible diseases. This improves the efficiency at which the disease infecting the given patient is identified and reduces the overall number of user interfaces and pages of information the provider or physician has to navigate through to identify the disease of interest associated with the patient.

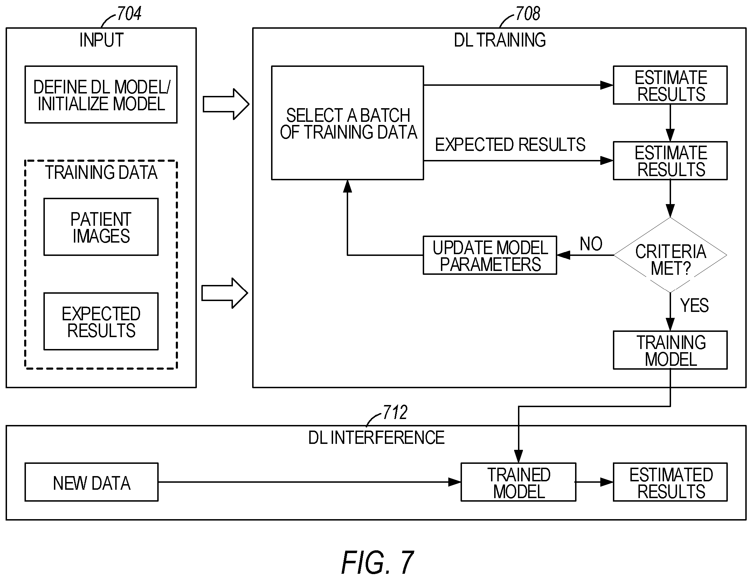

After receiving the multi-parametric input data associated with the patient and after identifying the disease associated with the patient, the treatment modality selection device 150 processes the multi-parametric input data to generate one or more metrics corresponding to a plurality of different modalities for treating the identified disease associated with the patient. For example, the treatment modality selection device 150 can use any one or a combination of heuristics, look-up tables, and machine learning models to process the multi-parametric input data to generate the metrics corresponding to the different modalities for treating the identified disease. Specifically, the treatment modality selection device 150 can generate various scores or weights for each possible or a subset of possible treatment modalities as the one or more metrics. The process for training the treatment modality selection device 150 to generate the one or more metrics for different treatment modalities is discussed in more detail below in connection with FIG. 7. Namely, the treatment modality selection device 150 can implement a machine learning technique (e.g., a neural network) that is trained based on training data to establish a relationship between a plurality of characteristics of pre-treatment planning associated with known patients associated with the disease, the modality of the plurality of different modalities used to treat the disease for each of the known patients, and a treatment result of each of the known patients. In some cases, the training data includes government or professional body regulations for treating the disease. In some cases, the training data includes reimbursement information of each of the plurality of different modalities.

In one example, the treatment modality selection device 150 can determine, based on processing the multi-parametric input data, that radiotherapy has a higher survival rate for treating the prostate cancer disease associated with the patient than chemotherapy or surgery. As a result, the treatment modality selection device 150 can assign a greater weight to the radiotherapy treatment modality than the chemotherapy and surgery modalities. Specifically, the treatment modality selection device 150 can associate radiotherapy with a score of 80, chemotherapy with a score of 15, and surgery with a score of 5 based on the past survival rate information of the different treatment modalities available for treating the identified prostate cancer disease.

In one example, the treatment modality selection device 150 can determine, based on processing the multi-parametric input data, that chemotherapy has a higher reimbursement value for treating the prostate cancer disease associated with the patient than radiotherapy. As a result, the treatment modality selection device 150 can assign a greater weight to the chemotherapy treatment modality than the radiotherapy modality. Specifically, the treatment modality selection device 150 can associate chemotherapy with a score of 60 and radiotherapy with a score of 40 based on the reimbursement value information of the different treatment modalities available for treating the identified prostate cancer disease.

In one example, the treatment modality selection device 150 can determine, based on processing the multi-parametric input data, that radiotherapy (1) has a higher survival rate for treating the prostate cancer disease associated with the patient than chemotherapy or surgery, (2) is associated with a higher reimbursement value and (3) is the governmental body or regulatory authority recommended course for treating prostate cancer. As a result, the treatment modality selection device 150 can assign a greater weight to the radiotherapy treatment modality than the chemotherapy and surgery modalities. Specifically, the treatment modality selection device 150 can associate radiotherapy with a score of 80, chemotherapy with a score of 10, and surgery with a score of 10 based on the past survival rate information of the different treatment modalities available for treating the identified prostate cancer disease, the reimbursement value and the governmental body or regulatory authority recommended course for treating prostate cancer.

In some embodiments, the treatment modality selection device 150 can select a given modality from the plurality of different modalities to treat the disease associated with the patient. In one implementation, the treatment modality selection device 150 automatically selects a highest ranked or highest scored treatment modality for treating the disease associated with the patient. In this case, no input is received from the provider or physician and the template or parameters of the given treatment modality are automatically generated and configured. In another implementation, the treatment modality selection device 150 presents a user interface to a provider or physician that identifies the disease associated with the patient and displays a list of the different modalities for treating the disease along with the corresponding metrics of each modality. Namely, the treatment modality selection device 150 can present a user interface that displays the possible treatment modalities in ranked order or according to the associated scores of each treatment modality.

Input can be received by the treatment modality selection device 150 from the provider or physician that selects a given one of the treatment modalities that are displayed. In some cases, the provider or physician selects a treatment modality that is associated with a lower score than other treatment modalities. Specifically, the provider or physician can select a treatment modality as the given modality for treating the patient that is ranked lower and displayed in a lower position in the list in the user interface. In such cases, a warning or prompt can be displayed to the physician or provider indicating that the selected treatment modality is not the highest ranked treatment modality. The warning or prompt can identify the highest ranked treatment modality. The warning or prompt can request confirmation from the provider or physician to proceed with the selected treatment modality as the given modality used to treat the patient. The warning or prompt can allow the provider or physician to select the highest ranked treatment modality instead of the treatment modality previously selected by the provider or physician. In these circumstances, if the provider or physician confirms proceeding with the lower ranked treatment modality as the modality used to treat the patient, the action can be recorded in a file associated with the provider or physician for performing quality assurance (QA).

In certain cases, the treatment modality selection device 150 can track the treatment results or survival rate of the treatment modality selected by the provider or physician. The treatment modality selection device 150 can compare the results or the survival rate with the survival rate or survival score of the highest ranked treatment modality that was not selected as the given modality to treat the patient. If the treatment modality selection device 150 detects that the survival rate or results are associated with a score that is lower than the survival rate or score of the highest ranked treatment modality by more than a threshold (e.g., by more than 10 percent), the treatment modality selection device 150 can trigger a prompt or warning to a supervisor or other healthcare monitoring facility. The prompt or warning can cause the supervisor or healthcare monitoring facility to audit the physician or provider. As an example, the treatment modality selection device 150 can recommend as the highest ranked treatment modality radiotherapy for treating prostate cancer. The provider can select a different treatment modality, such as chemotherapy, to treat the prostate cancer. The radiotherapy treatment modality can be associated with a survival rate score of 80%. The treatment modality selection device 150 can determine that as a result of treating the patient with chemotherapy instead of the highest ranked radiotherapy, the survival rate or result score was 40%. The treatment modality selection device 150 can determine that the survival rate or result score is more than 10% lower than the survival rate score of the radiotherapy treatment modality. As a result, the treatment modality selection device 150 can flag the file of the provider to cause the provider to be audited for failing to follow the recommended protocol or failing to follow the highest ranked treatment modality recommended by the treatment modality selection device 150.

In some embodiments, the treatment modality selection device 150 can configure one or more parameters of the given treatment modality, such as using some of the multi-parametric input data. For example, the treatment modality selection device 150 can retrieve from the database 124 a blank template associated with the given treatment modality that has been selected. The blank template can be processed to identify the fields of the template. The patient information derived from the multi-parametric input data, such as image features, EMR information, clinical information and so forth can be used to populate the fields of the template. The treatment modality selection device 150 can also specify in the template a type of the given treatment modality to apply. For example, if the selected treatment modality is radiotherapy, the treatment modality selection device 150 can specify the type of radiotherapy (e.g., a selection between external beam radiation therapy, gamma knife, stereotactic radiation therapy, and so forth) to use in the radiotherapy template. The treatment modality selection device 150 can select or specify the amount of dose a patient will receive based on intensity information contained in a PET scan of the region of interest.

The treatment modality selection device 150 can transmit the populated template to a suitable modality execution module, such as a radiotherapy planning system. Specifically, the treatment modality selection device 150 can transmit the populated radiotherapy template to the image processing device 112. The image processing device 112 can then apply one or more automated radiotherapy planning techniques to generate a suitable radiotherapy treatment plan based on the information included in the populated template. As mentioned above, once the radiotherapy treatment plan is generated by the image processing device 112, the image processing device 112 can instruct the radiation therapy device 130 to perform radiotherapy on the patient according to the parameters specified in the automatically generated radiotherapy treatment plan.

The treatment modality selection device 150 can receive outcome information from the modality execution module, such as the survival rate information, toxicity information, tumor reduction information or amount, and other outcome specific information. This output information can be used as new training data to update a model implemented by the treatment modality selection device 150. The treatment modality selection device 150 can update the model used to generate the one or more metrics of each of the plurality of different treatment modalities based on the outcome information received from the modality execution module. In this way, the machine learning technique implemented by the treatment modality selection device 150 can continuously or periodically be updated based on new training data generated based on application of a previously selected treatment modality and outcome information from the treatment modality.

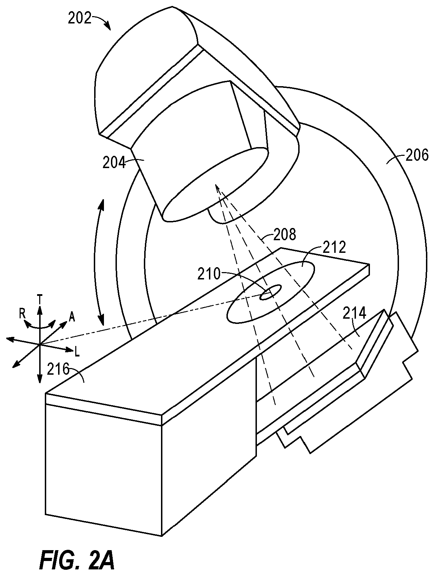

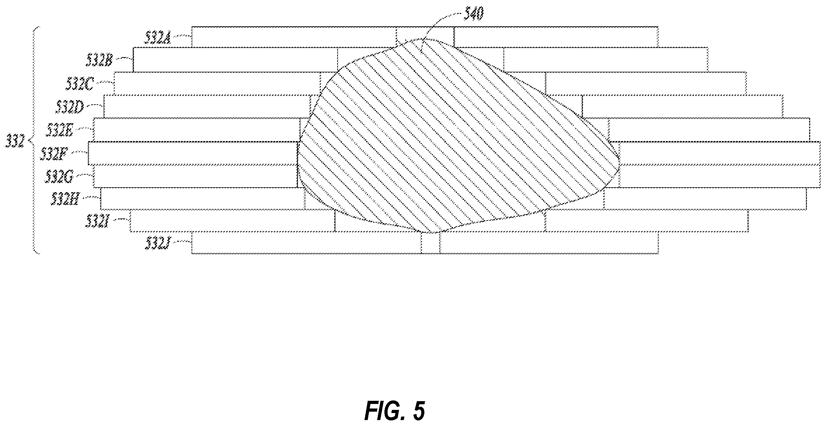

**FIG.** 2A illustrates an example radiation therapy device 202 that may include a radiation source, such as an X-ray source or a linear accelerator, a couch 216, an imaging detector 214, and a radiation therapy output 204. The radiation therapy device 202 may be configured to emit a radiation beam 208 to provide therapy to a patient. The radiation therapy output 204 can include one or more attenuators or collimators, such as an MLC as described in the illustrative embodiment of FIG. 5, below.

Referring back to FIG. 2A, a patient can be positioned in a region 212 and supported by the treatment couch 216 to receive a radiation therapy dose, according to a radiation therapy treatment plan. The radiation therapy output 204 can be mounted or attached to a gantry 206 or other mechanical support. One or more chassis motors (not shown) may rotate the gantry 206 and the radiation therapy output 204 around couch 216 when the couch 216 is inserted into the treatment area. In an embodiment, gantry 206 may be continuously rotatable around couch 216 when the couch 216 is inserted into the treatment area. In another embodiment, gantry 206 may rotate to a predetermined position when the couch 216 is inserted into the treatment area. For example, the gantry 206 can be configured to rotate the therapy output 204 around an axis ("*A*"). Both the couch 216 and the radiation therapy output 204 can be independently moveable to other positions around the patient, such as moveable in transverse direction ("*T*"), moveable in a lateral direction ("*L*"), or as rotation about one or more other axes, such as rotation about a transverse axis (indicated as "*R*"). A controller communicatively connected to one or more actuators (not shown) may control the couch's 216 movements or rotations in order to properly position the patient in or out of the radiation beam 208 according to a radiation therapy treatment plan. Both the couch 216 and the gantry 206 are independently moveable from one another in multiple degrees of freedom, which allows the patient to be positioned such that the radiation beam 208 can precisely target the tumor. The MLC may be integrated and included within gantry 206 to deliver the radiation beam 208 of a certain shape.

The coordinate system (including axes *A, T,* and *L*) shown in FIG. 2A can have an origin located at an isocenter 210. The isocenter 210 can be defined as a location where the central axis of the radiation beam 208 intersects the origin of a coordinate axis, such as to deliver a prescribed radiation dose to a location on or within a patient. Alternatively, the isocenter 210 can be defined as a location where the central axis of the radiation beam 208 intersects the patient for various rotational positions of the radiation therapy output 204 as positioned by the gantry 206 around the axis A. As discussed herein, the gantry angle corresponds to the position of gantry 206 relative to axis A, although any other axis or combination of axes can be referenced and used to determine the gantry angle.

Gantry 206 may also have an attached imaging detector 214. The imaging detector 214 is preferably located opposite to the radiation source, and in an embodiment, the imaging detector 214 can be located within a field of the therapy beam 208.

The imaging detector 214 can be mounted on the gantry 206 (preferably opposite the radiation therapy output 204), such as to maintain alignment with the therapy beam 208. The imaging detector 214 rotates about the rotational axis as the gantry 206 rotates. In an embodiment, the imaging detector 214 can be a flat panel detector (e.g., a direct detector or a scintillator detector). In this manner, the imaging detector 214 can be used to monitor the therapy beam 208 or the imaging detector 214 can be used for imaging the patient's anatomy, such as portal imaging. The control circuitry of radiation therapy device 202 may be integrated within system 100 or remote from it.

In an illustrative embodiment, one or more of the couch 216, the therapy output 204, or the gantry 206 can be automatically positioned, and the therapy output 204 can establish the therapy beam 208 according to a specified dose for a particular therapy delivery instance. A sequence of therapy deliveries can be specified according to a radiation therapy treatment plan, such as using one or more different orientations or locations of the gantry 206, couch 216, or therapy output 204. The therapy deliveries can occur sequentially, but can intersect in a desired therapy locus on or within the patient, such as at the isocenter 210. A prescribed cumulative dose of radiation therapy can thereby be delivered to the therapy locus while damage to tissue near the therapy locus can be reduced or avoided.

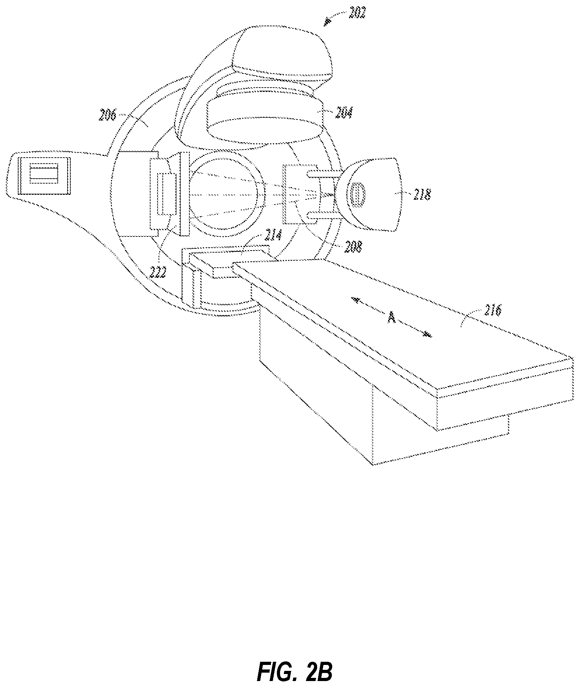

FIG. **2B** illustrates an example radiation therapy device 202 that may include a combined linac and an imaging system, such as can include a CT imaging system. The radiation therapy device 202 can include an MLC (not shown). The CT imaging system can include an imaging X-ray source 218, such as providing X-ray energy in a kiloelectron-Volt (keV) energy range. The imaging X-ray source 218 can provide a fan-shaped and/or a conical beam 208 directed to an imaging detector 222, such as a flat panel detector. The radiation therapy device 202 can be similar to the system described in relation to FIG. 2A, such as including a radiation therapy output 204, a gantry 206, a couch 216, and another imaging detector 214 (such as a flat panel detector). The X-ray source 218 can provide a comparatively lower-energy X-ray diagnostic beam, for imaging.

In the illustrative embodiment of FIG. 2B, the radiation therapy output 204 and the X-ray source 218 can be mounted on the same rotating gantry 206, rotationally separated from each other by 90 degrees. In another embodiment, two or more X-ray sources can be mounted along the circumference of the gantry 206, such as each having its own detector arrangement to provide multiple angles of diagnostic imaging concurrently. Similarly, multiple radiation therapy outputs 204 can be provided.

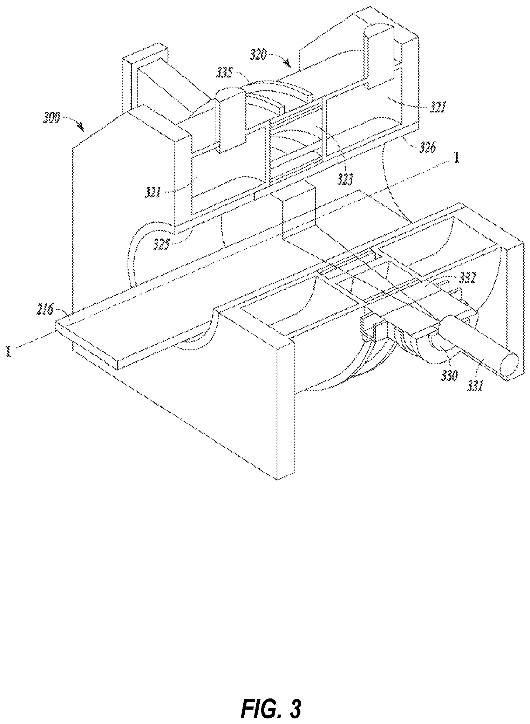

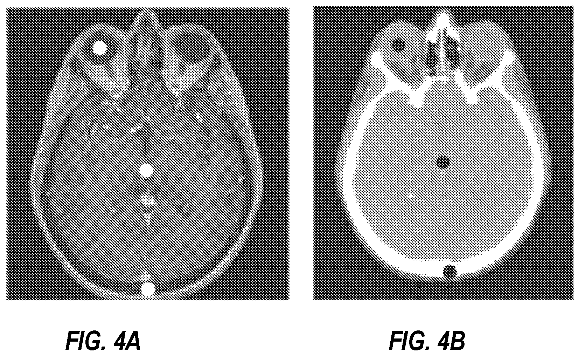

FIG. 3 depicts an example radiation therapy system 300 that can include combining a radiation therapy device 202 and an imaging system, such as a nuclear MR imaging system (e.g., known in the art as an MR-linac) consistent with the disclosed embodiments. As shown, system 300 may include a couch 216, an image acquisition device 320, and a radiation delivery device 330. System 300 delivers radiation therapy to a patient in accordance with a radiotherapy treatment plan. In some embodiments, image acquisition device 320 may correspond to image acquisition device 132 in FIG. 1 that may acquire origin images of a first imaging modality (e.g., MRI image shown in FIG. 4A) or destination images of a second imaging modality (e.g., CT image shown in FIG. 4B).

Couch 216 may support a patient (not shown) during a treatment session. In some implementations, couch 216 may move along a horizontal translation axis (labelled "1"), such that couch 216 can move the patient resting on couch 216 into and/or out of system 300. Couch 216 may also rotate around a central vertical axis of rotation, transverse to the translation axis. To allow such movement or rotation, couch 216 may have motors (not shown) enabling the couch to move in various directions and to rotate along various axes. A controller (not shown) may control these movements or rotations in order to properly position the patient according to a treatment plan.

In some embodiments, image acquisition device 320 may include an MRI machine used to acquire 2D or 3D MRI images of the patient before, during, and/or after a treatment session. Image acquisition device 320 may include a magnet 321 for generating a primary magnetic field for magnetic resonance imaging. The magnetic field lines generated by operation of magnet 321 may run substantially parallel to the central translation axis I. Magnet 321 may include one or more coils with an axis that runs parallel to the translation axis I. In some embodiments, the one or more coils in magnet 321 may be spaced such that a central window 323 of magnet 321 is free of coils. In other embodiments, the coils in magnet 321 may be thin enough or of a reduced density such that they are substantially transparent to radiation of the wavelength generated by radiotherapy device 330. Image acquisition device 320 may also include one or more shielding coils, which may generate a magnetic field outside magnet 321 of approximately equal magnitude and opposite polarity in order to cancel or reduce any magnetic field outside of magnet 321. As described below, radiation source 331 of radiotherapy device 330 may be positioned in the region where the magnetic field is cancelled, at least to a first order, or reduced.

Image acquisition device 320 may also include two gradient coils 325 and 326, which may generate a gradient magnetic field that is superposed on the primary magnetic field. Coils 325 and 326 may generate a gradient in the resultant magnetic field that allows spatial encoding of the protons so that their position can be determined. Gradient coils 325 and 326 may be positioned around a common central axis with the magnet 321 and may be displaced along that central axis. The displacement may create a gap, or window, between coils 325 and 326. In embodiments where magnet 321 can also include a central window 323 between coils 325 and 326, the two windows may be aligned with each other.

In some embodiments, image acquisition device 320 may be an imaging device other than an MRI, such as an X-ray, a CT, a CBCT, a spiral CT, a PET, a SPECT, an optical tomography, a fluorescence imaging, ultrasound imaging, radiotherapy portal imaging device, or the like. As would be recognized by one of ordinary skill in the art, the above description of image acquisition device 320 concerns certain embodiments and is not intended to be limiting.

Radiotherapy device 330 may include the radiation source 331, such as an X-ray source or a linac, and an MLC 332 (shown below in FIG. 5). Radiotherapy device 330 may be mounted on a chassis 335. One or more chassis motors (not shown) may rotate chassis 335 around couch 216 when couch 216 is inserted into the treatment area. In an embodiment, chassis 335 may be continuously rotatable around couch 216, when couch 216 is inserted into the treatment area. Chassis 335 may also have an attached radiation detector (not shown), preferably located opposite to radiation source 331 and with the rotational axis of chassis 335 positioned between radiation source 331 and the detector. Further, device 330 may include control circuitry (not shown) used to control, for example, one or more of couch 216, image acquisition device 320, and radiotherapy device 330. The control circuitry of radiotherapy device 330 may be integrated within system 300 or remote from it.

During a radiotherapy treatment session, a patient may be positioned on couch 216. System 300 may then move couch 216 into the treatment area defined by magnet 321, coils 325 and 326, and chassis 335. Control circuitry may then control radiation source 331, MLC 332, and the chassis motor(s) to deliver radiation to the patient through the window between coils 325 and 326 according to a radiotherapy treatment plan.

FIG. 2A, FIG. 2B, and FIG. 3 illustrate generally embodiments of a radiation therapy device configured to provide radiotherapy treatment to a patient, including a configuration where a radiation therapy output can be rotated around a central axis (e.g., an axis "A"). Other radiation therapy output configurations can be used. For example, a radiation therapy output can be mounted to a robotic arm or manipulator having multiple degrees of freedom. In yet another embodiment, the therapy output can be fixed, such as located in a region laterally separated from the patient, and a platform supporting the patient can be used to align a radiation therapy isocenter with a specified target locus within the patient.

As discussed above, radiation therapy devices described by FIG. 2A, FIG. 2B, and FIG. 3 include an MLC for shaping, directing, or modulating an intensity of a radiation therapy beam to the specified target locus within the patient. FIG. 5 illustrates an example MLC 332 that includes leaves 532A through 532J that can be automatically positioned to define an aperture approximating a tumor 540 cross section or projection. The leaves 532A through 532J permit modulation of the radiation therapy beam. The leaves 532A through 532J can be made of a material specified to attenuate or block the radiation beam in regions other than the aperture, in accordance with the radiation treatment plan. For example, the leaves 532A through 532J can include metallic plates, such as comprising tungsten, with a long axis of the plates oriented parallel to a beam direction and having ends oriented orthogonally to the beam direction (as shown in the plane of the illustration of FIG. 2A). A "state" of the MLC 332 can be adjusted adaptively during a course of radiation therapy treatment, such as to establish a therapy beam that better approximates a shape or location of the tumor 540 or another target locus. This is in comparison to using a static collimator configuration or as compared to using an MLC 332 configuration determined exclusively using an "offline" therapy planning technique. A radiation therapy technique using the MLC 332 to produce a specified radiation dose distribution to a tumor or to specific areas within a tumor can be referred to as IMRT.

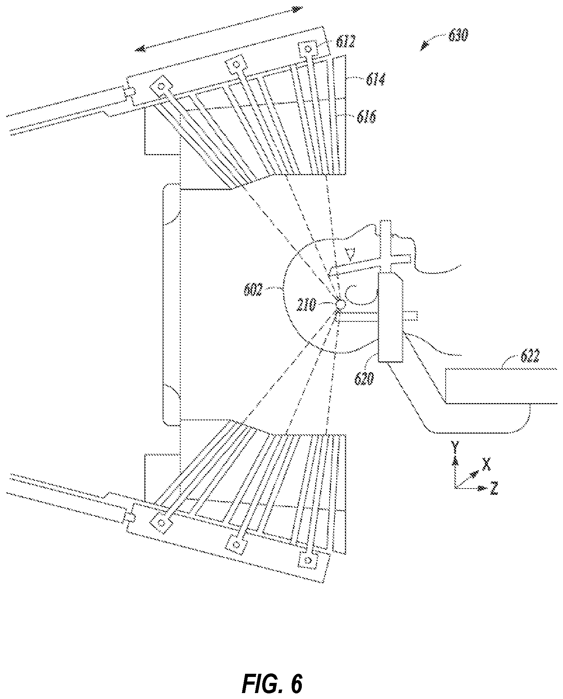

**FIG. 6** illustrates an embodiment of another type of radiotherapy device 630 (e.g., a Leksell Gamma Knife), according to some embodiments of the present disclosure. As shown in FIG. 6, in a radiotherapy treatment session, a patient 602 may wear a coordinate frame 620 to keep stable the patient's body part (e.g., the head) undergoing surgery or radiotherapy. Coordinate frame 620 and a patient positioning system 622 may establish a spatial coordinate system, which may be used while imaging a patient or during radiation surgery. Radiotherapy device 630 may include a protective housing 614 to enclose a plurality of radiation sources 612. Radiation sources 612 may generate a plurality of radiation beams (e.g., beamlets) through beam channels 616. The plurality of radiation beams may be configured to focus on an isocenter 210 from different directions. While each individual radiation beam may have a relatively low intensity, isocenter 210 may receive a relatively high level of radiation when multiple doses from different radiation beams accumulate at isocenter 210. In certain embodiments, isocenter 210 may correspond to a target under surgery or treatment, such as a tumor.

**FIG.** 7 illustrates an example flow diagram for deep learning, where a deep learning model (or a machine learning model), such as a deep convolutional neural network (DCNN), can be trained and used to determine one or more metrics of a plurality of treatment modalities for treating a disease associated with a patient. A treatment modality can be selected from the plurality of treatment modalities and can be used to select a template to execute a treatment plan for the treatment modality. As an example, the treatment modality that is selected can include a type of radiotherapy including one or any combination of external beam radiation therapy, gamma knife therapy, stereotactic radiation therapy, and/or proton therapy which can be used to generate a radiotherapy template. The radiotherapy template is then processed to produce or update a radiotherapy treatment plan or configure one or more radiotherapy treatment parameters.

Inputs 704 can include a defined deep learning model (which can include one or more sub-networks or one or more individual and independent machine learning models) having an initial set of values (e.g., nominal startup values or random DCNN parameter values) and training data. The training data can include multi-parametric input data, such as any combination of one or more image features, one or more features derived from an image, clinical data, EMR information associated with the patient, and outcome metrics. The training data can include characteristics of pre-treatment planning associated with a plurality of known patients, a treatment modality used to treat the disease for the plurality of known patients, and the treatment result of the plurality of known patients. The training data can also include government or professional body regulations for treating the disease, and reimbursement information of each of the plurality of different modalities. The training data can also include data sets, where each data set includes a first set of characteristics of pre-treatment planning associated with a given known patient (e.g., the image features, features derived from one or more images of the known patient, clinical data associated with the known patient, and EMR information associated with the known patient), a modality used to treat the disease for the given known patient, and the treatment result (outcome metric) of the given known patient. The training data can include multiple of these paired data sets for multiple patients.

The clinical data can include any combination of a Gleason score, a PSA score, previous surgical intervention data, previous radiation treatments information, previous systemic therapies information, proximity to organs at risk data, obstructive symptoms information, presence of specific heritable pathogenic variants information, laterality information, morphology information, ethnicity information, gene panels information, genetic mutations information, ultrasound data, endoscopy data, physical examination results, urine test information, blood test information, biopsy data, HPV infection, gender, age, Epstein-Barr infection information, clear surgical margins data, Eastern Cooperative Oncology Group (ECOG) status information (which can describe a patient's level of functioning in terms of their ability to care for themselves, daily activity and physical ability ranging from grades 0-5, in which: grade 0 defines a patient who is fully active, able to carry on all pre-disease performance without restriction, grade 1 defines a patient restricted in their physically strenuous activity but ambulatory and able to carry out work of a light or sedentary nature; grade 2 defines a patient who is ambulatory and capable of all selfcare but unable to carry out any work activities; grade 3 defines a patient who is capable of only limited selfcare and is confined to a bed or chair more than 50% of waking hours; grade 4 defines a completely disabled patient; and grade 5 is a dead patient), radiosensitising agent conditions information, collagen vascular disease information, Non-invasive ductal carcinoma in situ information, BMI information, level of T-Cells in a body information, pregnancy status information, suitability of surgery information, and PET staging information.

The deep learning model can include one or more neural networks (referred to as sub-networks), such as a DCNN. The deep learning network can be trained on the training data to establish a relationship between a plurality of characteristics of pre-treatment planning associated with known patients associated with the disease, the modality of the plurality of different modalities used to treat the disease for each of the known patients, and a treatment result of each of the known patients. In one embodiment, the deep learning network is trained in an end-to-end manner in which all of the sub-networks are trained simultaneously by being applied to a same set or batch of training data and minimizing a set of cost functions. In another embodiment, one or more of the sub-networks of the DCNN are trained separately and independently in sequence by minimizing a set of cost functions associated with each particular sub-network. In some cases, each sub-network of the DCNN is disease specific such that it is trained based on a respective set of the training data associated with a specific disease. In this way, different sub-networks of the DCNN are configured to estimate a set of modalities for treating different diseases associated with the respective sub-networks. For example, a first sub-network can be configured to estimate a set of modalities for treating breast cancer (e.g., a first disease) and a second sub-network can be configured to estimate a set of modalities for treating prostate cancer (e.g., a second disease).

The training data can include medical images of the known patients that can include images of an anatomy, CT images, PET images, or MRI images. When trained, the deep learning network can produce an estimate of a set of modalities for treating the disease and their respective metrics (e.g., ranking or weights). The expected results can include the actual modality used to treat a given known patient and the corresponding survival and/or toxicity information.

During training of deep learning (DL) model 708, a batch of training data can be selected from the pairs of the training data of known patients and expected results (e.g., the corresponding actual modalities used to treat the patients and/or the modalities specified by the governmental or professional bodies as recommended modalities for treating the disease and/or the reimbursement values or information associated with different treatment modalities for treating the disease). The selected training data can include a portion of the training data corresponding to a given known patient of the known patients, the portion of the training data comprising characteristics of pre-treatment planning associated with the given known patient, the modality used to treat the disease for the given known patient, and the treatment result of the given known patient. In the case of end-to-end training, the batch of training data can be processed by all of the sub-networks of the DL model 708 simultaneously.

The deep learning model 708 can be applied to the selected training data to provide estimated results (e.g., estimated set of modalities for treating the disease of the given known patient), which can then be compared to the expected results (e.g., the actual modalities used to treat the patients, the treatment results, survival rate, toxicity information and/or the modalities specified by the governmental or professional bodies as recommended modalities for treating the disease and/or the reimbursement values or information associated with different treatment modalities for treating the disease) to compute a difference or deviation that can provide an indication of training errors. The difference or deviation can be used to compute a loss function. The loss function can be computed based on a deviation parameter and a treatment result parameter. The deviation parameter can be determined based a result of comparing the modality used to treat the disease for the given known patient with the estimated set of modalities. The treatment result parameter can be determined based on the treatment result (e.g., the survival rate and/or toxicity information) of the given known patient. In some cases, the loss function further includes a reimbursement parameter specifying a level of reimbursement of each of the modalities for treating the disease. In some cases, the loss function further includes a government or professional body regulations parameter specifying a modality for treating the disease given a set of characteristics.

In some cases, the DCNN is trained to generate a weight for each of the set of modalities for treating the disease of the given known patient. The weight can be generated based on the computed loss function. For example, the DCNN can compute the loss function for each of a plurality of the estimated treatment modalities. The DCNN can compare the value of the loss or the errors associated with each selected treatment modality and can rank the treatment modalities or generate the metrics of each treatment modality as a function of the amount of the loss or errors. For example, if a first treatment modality is associated with a lower loss (e.g., because the survival rate indicated by the treatment result parameter is greater and the toxicity information is lower) than a second treatment modality, the DCNN can rank the first treatment modality higher than the second treatment modality.

The errors or result of computing the loss function can be used during a procedure called backpropagation to update the parameters of the deep learning network (e.g., layer node weights and biases of each or of certain sub-networks of the model 708), in order to reduce or minimize errors during subsequent trials. The errors or result of computing the loss function can be compared to predetermined criteria, such as proceeding to a sustained minimum for a specified number of training iterations. If the errors or result of computing the loss function do not satisfy the predetermined criteria, then model parameters of the deep learning model 708 can be updated using backpropagation, and another batch of training data can be selected from the other sets of training data (of the same patient or other patients) and expected results for another iteration of deep learning model training. If the errors or result of computing the loss function satisfy the predetermined criteria, then the training can be ended, and the trained model 708 can then be used during a deep learning testing or inference stage 712 to generate one or more metrics for different treatment modalities for treating a disease associated with a new patient. The trained model 708 can receive new multi-parametric data for the new patient and can provide estimated results (e.g., the estimated set of modalities for treating the disease of the new patient).

After updating the parameters of the DCNN, the iteration index can be incremented by a value of one. The iteration index can correspond to a number of times that the parameters of the DCNN have been updated. Stopping criteria can be computed, and if the stopping criteria are satisfied, then the DCNN model can be saved in a memory, such as a memory device of treatment modality selection device 150, and the training can be halted. If the stopping criteria are not satisfied, then the training can continue by obtaining another batch of training data from the same training subject or another training subject. In an embodiment, the stopping criteria can include a value of the iteration index (e.g., the stopping criteria can include whether the iteration index is greater than or equal to a determined maximum number of iterations). In an embodiment, the stopping criteria can include an accuracy of the output treatment modalities or metrics of the different treatment modalities (e.g., the stopping criteria can include whether the difference between the output treatment modalities or metrics of the different treatment modalities and the actual modalities used to treat the patients and/or the modalities specified by the governmental or professional bodies as recommended modalities for treating the disease and/or the reimbursement values or information associated with different treatment modalities for treating the disease in the batch of training data is smaller than a threshold).

In some embodiments, the DL model 708 is re-trained to estimate a set of treatment modalities and corresponding metrics for a different disease. Namely, the DL model 708 can be trained to generate a set of modalities for treating a second disease based on additional training data comprising a plurality of characteristics of pre-treatment planning associated with known patients associated with the second disease, the modality of the plurality of different modalities used to treat the second disease for each of the known patients, and a treatment result of each of the known patients associated with the second disease. The steps recited above for training the DL model 708 can be repeated for each set or batch of training data associated with different diseases.

After the DL model 708 is trained, multi-parametric input data representing data associated with a patient or an anatomy can be received, such as from the database 124. The multi-parametric input data can include any combination of one or more image features, one or more features derived from an image, clinical data, and EMR information associated with the patient. The trained DCNN model can be received from a network, such as the network 120, or from a memory, such as the memory device of the treatment modality selection device 150. The trained DCNN can be used to determine the one or more metrics corresponding to a plurality of different modalities for treating the disease associated with the patient based on the multi-parametric input data. As an example, the clinical data associated with the patient can include any combination of a Gleason score, a prostate-specific antigen (PSA) score, previous surgical intervention data, previous radiation treatments information, previous systemic therapies information, proximity to organs at risk data, obstructive symptoms information, presence of specific heritable pathogenic variants information, laterality information, morphology information, ethnicity information, gene panels information, genetic mutations information, ultrasound data, endoscopy data, physical examination results, urine test information, blood test information, biopsy data, Human papillomavirus infection (HPV) infection, gender, age, Epstein-Barr infection information, clear surgical margins data, Eastern Cooperative Oncology Group (ECOG) status information, radiosensitising conditions information, collagen vascular disease information, Non-invasive ductal carcinoma in situ information, body mass index (BMI) information, level of T-Cells in a body information, pregnancy status information, suitability of surgery information, and positron emission tomography (PET) staging information.

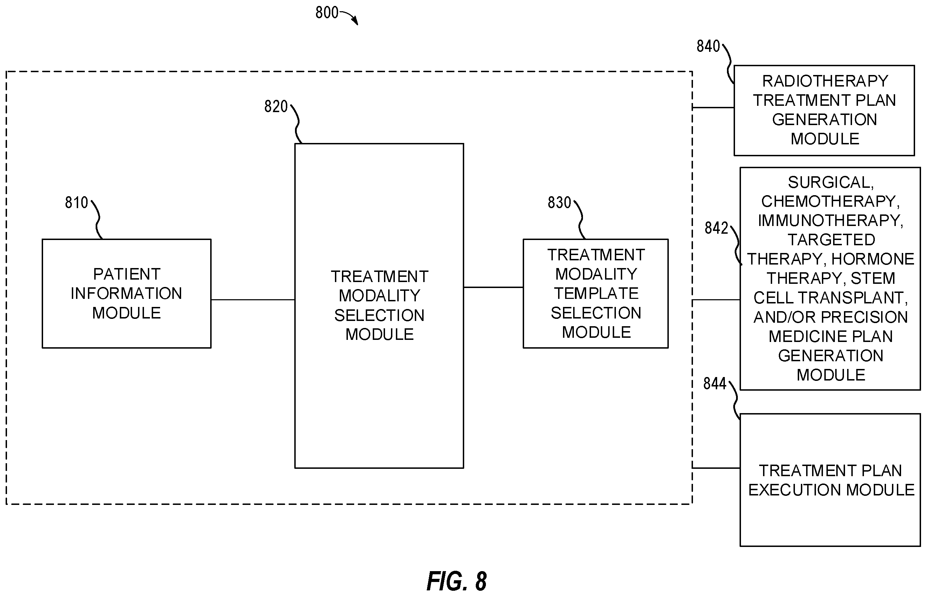

**FIG. 8** illustrates an example of a treatment modality selection system 800, according to some embodiments of the present disclosure. The treatment modality selection system 800 includes a patient information module 810, a treatment modality selection module 820, a treatment modality template selection module 830, a radiotherapy treatment plan generation module 840, a surgical, chemotherapy, immunotherapy, targeted therapy, hormone therapy, stem cell transplant, and/or precision medicine plan generation module 842, and a treatment plan execution module 844. The components of the treatment modality selection system 800 can be implemented by a single processing circuitry or server or can be distributed among multiple processing circuitries or servers. The treatment modality selection system 800 can be included in or implement the treatment modality selection device 150 and/or the image processing device 112.

The patient information module 810 is configured to access patient records for a given patient. For example, the patient information module 810 can access EMR information stored in the database 124 and imaging information stored in the medical images 146. The patient information module 810 can also access a questionnaire filled out by the given patient (or provider/physician) to determine additional more recent information about the given patient than what is stored in the database 124. The patient information module 810 can detect differences between data input in the questionnaire and the data stored in the database 124. The patient information module 810 can update the data stored in the database 124 for the given patient with the more recent data input in the questionnaire. The patient information module 810 can receive input from a provider or physician including health related information about the given patient. For example, while the given patient is seeing the provider or physician, the provider or physician can directly input information provided by the patient using a user interface to the patient information module 810. In some cases, the patient information module 810 includes a voice recognition system or speech detection system and can automatically populate information for the given patient based on speech input received from the given patient.

The patient information module 810 can also access one or more image features, one or more features derived from an image and clinical data. The one or more image features can include a size, volume, or intensity of a region of interest, such as a prostate. The one or more features derived from an image can include radiomics features including texture and gradients of the one or more image features. The clinical data can include staging, genomics, and/or one or more tumor specific features, such as: a Gleason score, or a prostate-specific antigen (PSA) score.

After collecting all the information about the given patient, the patient information module 810 generates a first portion of multi-parametric input data. The patient information module 810 can access governmental body information or professional body information about various diseases, including reimbursement information and outcome metrics for different modes of treatments for the various diseases. This information can be accessed over the Internet or from a dedicated source. For example, the patient information module 810 can access one or more outcome metrics, such as: toxicities, disease-free survival information for previous patients with the disease, or reimbursement information for a plurality of different treatment modalities. For example, the multi-parametric input data can include a table or list of diseases along with their respective survival rates for different treatment modalities. The patient information module 810 can also access such information on a geographical region basis. Namely, the patient information module 810 can associate some or all of the information about the outcome metrics and reimbursement with different geographical regions. The patient information module 810 can generate a second portion of the multi-parametric input data using the governmental body information or professional body information about various diseases, including reimbursement information and outcome metrics for different modes of treatments for the various diseases.

The patient information module 810 provides the first and second portions of the multi-parametric input data to the treatment modality selection module 820. In some cases, the treatment modality selection module 820 presents a user interface to the provider/physician to select a given disease from a list of possible diseases that infect or are associated with the given patient. The treatment modality selection module 820 processes the multi-parametric input data received from the patient information module 810 using a given model. For example, the treatment modality selection module 820 implements a model 900 (F'IG. 9). The model 900 receives the multi-parametric input data 910 and applies the multi-parametric input data 910 to different treatment processing modules 920 to generate respective treatment modalities. Each modality output by the treatment processing modules 920 can be associated with a different outcome metric. The output module 930 receives all the different treatment modalities with the different outcome metrics from the treatment processing modules 920 and generates a ranking of the different treatment modalities.

For **example, each** treatment processing modules 920 is configured to process the same set of multi-parametric input data 910 using a different set of rules. In some cases, each treatment processing modules 920 receives a different subset of the set of multi-parametric input data 910 with the information pertinent to the given treatment processing modules 920. For example, one of treatment processing modules 920 can process the set of multi-parametric input data 910 independently of reimbursement information for the given disease while another processes the set of multi-parametric input data 910 inclusive of the reimbursement information for the given disease. In such cases, the set of multi-parametric input data 910 supplied to one of the treatment processing modules 920 can include the reimbursement information and that supplied to another may exclude the reimbursement information.

Each of the treatment processing modules 920 can be associated with a different weight, such that the output of the treatment modality of the given treatment processing module 920 is weighted by the respective weight of the treatment processing module 920. For example, a first of the treatment processing modules 920 is configured to process the multi-parametric input data 910 using a machine learning technique to estimate one or more treatment modality. The first treatment processing module 920 is associated with a weight of 0.9 which can be used as an adjustment factor for each of the estimated one or more treatment modalities. As another example, a second of the treatment processing modules 920 is configured to process the multi-parametric input data 910 using a look-up table. The look-up table can be provided by a governmental body or professional body that associates different patient information and different diseases with respective treatment modalities. The second treatment processing module 920 is associated with a weight of 0.7 which can be used as an adjustment factor for each of the treatment modalities provided by the second treatment processing module 920. In this way, if the second treatment processing module 920 provides a first treatment modality and the first treatment processing module 920 provides a different second treatment modality, the output module 930 may rank the second treatment modality higher than the first treatment modality because the second treatment processing module 920 is associated with a greater weight (e.g., 0.9) than the weight (e.g., 0.7) associated with the first treatment processing module 920.

In some implementations, a third of the treatment processing modules 920 is configured to process the multi-parametric input data 910 using a reimbursement value table. The reimbursement value table can be provided by a governmental body that associates different patient information, reimbursement values, and different diseases with respective treatment modalities. The third treatment processing module 920 can select to output the treatment modality that is associated with a highest reimbursement value for the combination of patient information and disease. The third treatment processing module 920 is associated with a weight of 0.8 which can be used as an adjustment factor for each of the treatment modalities provided by the third treatment processing module 920.

In one example, the output module 930 of the treatment modality selection module is presented on a graphical user interface. The graphical user interface provides the ranked list of the different treatment modalities for treating the given disease. The graphical user interface visually identifies the top ranked or recommended treatment modality. The treatment modality selection module 820 receives user input from the provider or physician via the graphical user interface that selects a given treatment modality from the ranked list of treatment modalities. In some cases, the treatment modality selection module 820 stores an indication that the selected treatment modality is not the highest ranked treatment modality in response to detecting that the treatment modality selected by the user input is not the recommended treatment modality generated by the treatment modality selection module 820.

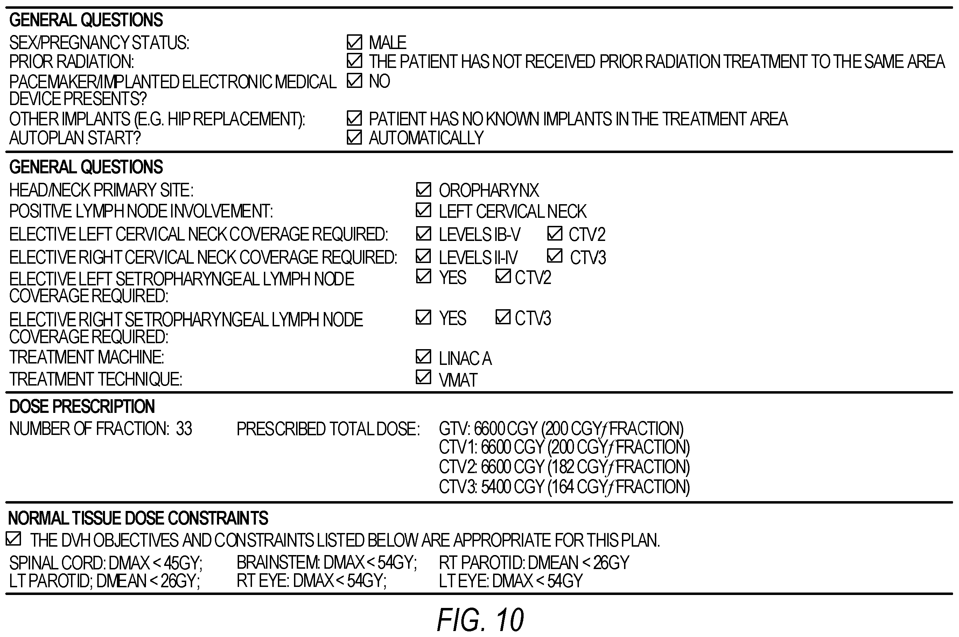

The selected treatment modality is provided to the treatment modality template selection module 830. The treatment modality template selection module 830 also receives at least a portion of the multi-parametric data from the patient information module 810. The treatment modality template selection module 830 retrieves a blank or partially filled template associated with the selected treatment modality. The treatment modality template selection module 830 automatically populates the template with the information received from the patient information module 810 and/or additional inputs received from the provider/physician. For example, the treatment modality template selection module 830 can select a radiotherapy treatment template in response to determining that the selected treatment modality includes radiotherapy. An example radiotherapy treatment template is shown in FIG. 10. The treatment modality template selection module 830 can populate the type of disease and the radiotherapy treatment parameters (e.g., location of the region of interest, type of radiotherapy, and dose information) into the template.

The treatment modality template selection module 830 transmits the completed treatment modality template to the corresponding treatment execution module. For example, if the treatment modality template corresponds to radiotherapy, the treatment modality template selection module 830 transmits the completed treatment modality template to the radiotherapy treatment plan generation module 840. The radiotherapy treatment plan generation module 840 can apply various heuristics and machine learning techniques to automatically generate a radiotherapy treatment plan that includes radiotherapy device parameters (e.g., number of fractions, segmentations, dose maps, and so forth) for executing the radiotherapy treatment plan. As another example, if the treatment modality template corresponds to surgery, the treatment modality template selection module 830 transmits the completed treatment modality template to the surgical, chemotherapy, immunotherapy, targeted therapy, hormone therapy, stem cell transplant, and/or precision medicine plan generation module 842. Module 842 can apply various heuristics and machine learning techniques to automatically generate a surgical plan for executing the surgical treatment plan. As another example, if the treatment modality template corresponds to any other type of treatment modality that may not be included in the module 842 (e.g., chemotherapy, immunotherapy, targeted therapy, hormone therapy, a stem cell transplant, precision medicine, any combination of surgery, radiotherapy, chemotherapy, immunotherapy, targeted therapy, hormone therapy, a stem cell transplant, precision medicine, and preventing performance of any treatment), the treatment modality template selection module 830 transmits the completed treatment modality template to the treatment plan execution module 844. The treatment plan execution module 844 can apply various heuristics and machine learning techniques to automatically generate a treatment plan for executing the type of treatment specified by the treatment modality template.

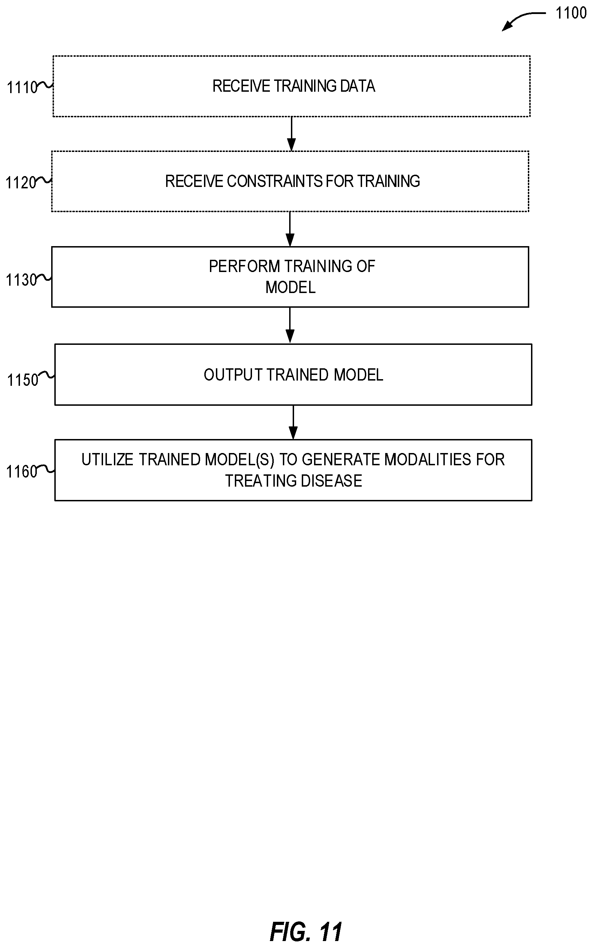

**FIG. 11** is a flowchart illustrating example operations of the treatment modality selection device 150 in performing process 1100, according to example embodiments. The process 1100 may be embodied in computer-readable instructions for execution by one or more processors such that the operations of the process 1100 may be performed in part or in whole by the functional components of the image processing device 112 and/or the treatment modality selection device 150; accordingly, the process 1100 is described below by way of example with reference thereto. However, in other embodiments, at least some of the operations of the process 1100 may be deployed on various other hardware configurations. The process 1100 is therefore not intended to be limited to the image processing device 112 and can be implemented in whole, or in part, by any other component. Some or all of the operations of process 1100 can be in parallel, out of order, or entirely omitted.

At operation 1110, treatment modality selection device 150 receives training data. For example, image processing device 112 receives training data, which may include paired training data sets (e.g., input-output training pairs).

At operation 1120, treatment modality selection device 150 receives one or more cost functions for training the model.

At operation 1130, treatment modality selection device 150 performs training of the model based on the received training data and one or more cost functions.

At operation 1150, treatment modality selection device 150 outputs the trained model. For example, treatment modality selection device 150 outputs the trained model to operate on a new set of multi-parametric input data to generate one or more modalities for treating a disease.

At operation 1160, treatment modality selection device 150 utilizes the trained model to generate one or more modalities for treating a disease.

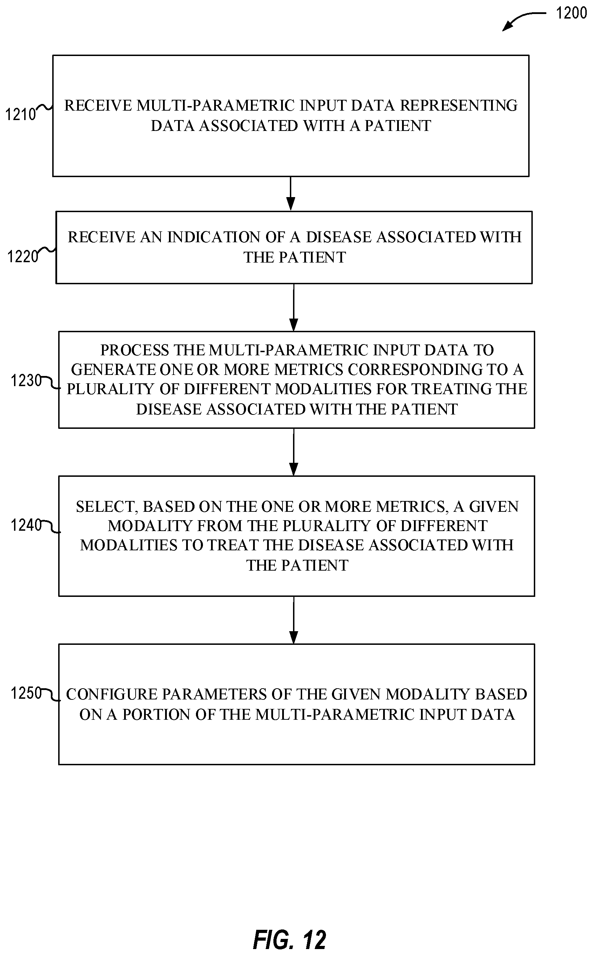

FIG. **12** is a flowchart illustrating example operations of the treatment modality selection device 150 in performing process 1200, according to example embodiments. The process 1200 may be embodied in computer-readable instructions for execution by one or more processors such that the operations of the process 1200 may be performed in part or in whole by the functional components of the treatment modality selection device 150; accordingly, the process 1200 is described below by way of example with reference thereto. However, in other embodiments, at least some of the operations of the process 1200 may be deployed on various other hardware configurations. The process 1200 is therefore not intended to be limited to the image processing device 112 and can be implemented in whole, or in part, by any other component. Some or all of the operations of process 1200 can be in parallel, out of order, or entirely omitted.

At operation 1210, treatment modality selection device 150 receives multi-parametric input data representing data associated with a patient, as discussed above.

At operation 1220, treatment modality selection device 150 receives an indication of a disease associated with the patient, as discussed above.

At operation 1230, treatment modality selection device 150 processes the multi-parametric input data to generate one or more metrics corresponding to a plurality of different modalities for treating the disease associated with the patient, as discussed above.

At operation 1240, treatment modality selection device 150 selects, based on the one or more metrics, a given modality from the plurality of different modalities to treat the disease associated with the patient;, as discussed above.

At operation 1250, treatment modality selection device 150 configures parameters of the given modality based on a portion of the multi-parametric input data, as discussed above.

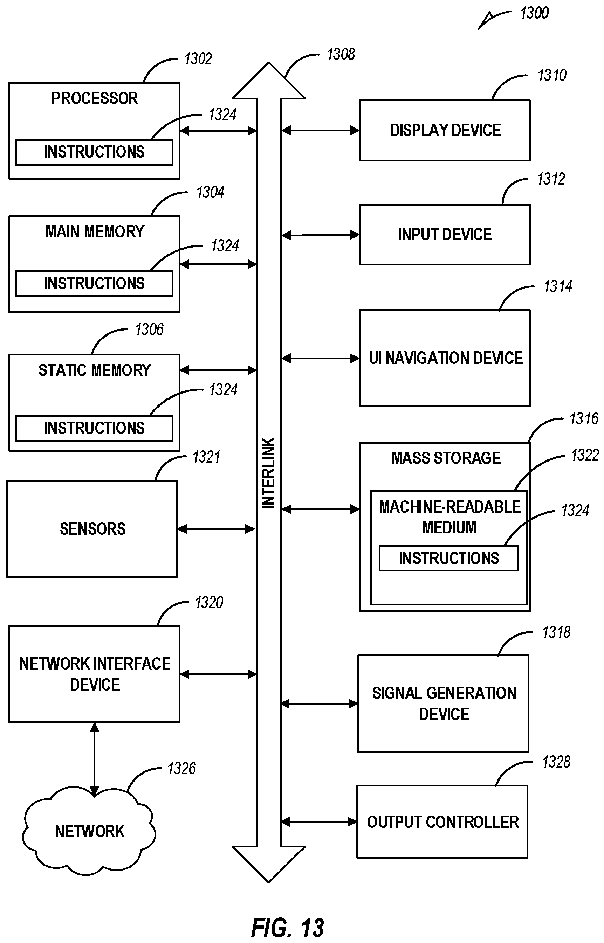

**FIG. 13** illustrates a block diagram of an embodiment of a machine 1300 on which one or more of the methods as discussed herein can be implemented. In one or more embodiments, one or more items of the image processing device 112 can be implemented by the machine 1300. In alternative embodiments, the machine 1300 operates as a standalone device or may be connected (e.g., networked) to other machines. In one or more embodiments, the image processing device 112 can include one or more of the items of the machine 1300. In a networked deployment, the machine 1300 may operate in the capacity of a server or a client machine in server-client network environment, or as a peer machine in a peer-to-peer (or distributed) network environment. The machine may be a personal computer (PC), a tablet PC, a set-top box (STB), a Personal Digital Assistant (PDA), a cellular telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

The example machine 1300 includes processor 1302 (e.g., a CPU, a GPU, an ASIC, circuitry, such as one or more transistors, resistors, capacitors, inductors, diodes, logic gates, multiplexers, buffers, modulators, demodulators, radios (e.g., transmit or receive radios or transceivers), sensors 1321 (e.g., a transducer that converts one form of energy (e.g., light, heat electrical, mechanical, or other energy) to another form of energy), or the like, or a combination thereof), a main memory 1304 and a static memory 1306, which communicate with each other via a bus 1308. The machine 1300 (e.g., computer system) may further include a video display unit 1310 (e.g., a liquid crystal display (LCD) or a cathode ray tube (CRT)). The machine 1300 also includes an alphanumeric input device 1312 (e.g., a keyboard), a user interface (UI) navigation device 1314 (e.g., a mouse), a disk drive or mass storage unit 1316, a signal generation device 1318 (e.g., a speaker), and a network interface device 1320.

The disk drive or mass storage unit 1316 includes a machine-readable medium 1322 on which is stored one or more sets of instructions and data structures (e.g., software) 1324 embodying or utilized by any one or more of the methodologies or functions described herein. The instructions 1324 may also reside, completely or at least partially, within the main memory 1304 and/or within the processor 1302 during execution thereof by the machine 1300, the main memory 1304 and the processor 1302 also constituting machine-readable media.

The machine 1300 as illustrated includes an output controller 1328. The output controller 1328 manages data flow to/from the machine 1300. The output controller 1328 is sometimes called a device controller, with software that directly interacts with the output controller 1328 being called a device driver.

While the machine-readable medium 1322 is shown in an embodiment to be a single medium, the term "machine-readable medium" may include a single medium or multiple media (e.g., a centralized or distributed database, and/or associated caches and servers) that store the one or more instructions or data structures. The term "machine-readable medium" shall also be taken to include any tangible medium that is capable of storing, encoding or carrying instructions for execution by the machine and that cause the machine to perform any one or more of the methodologies of the present disclosure, or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including by way of example semiconductor memory devices, e.g., Erasable Programmable Read-Only Memory (EPROM), EEPROM, and flash memory devices; magnetic disks such as internal hard disks and removable disks; magneto-optical disks; and CD-ROM and DVD-ROM disks.

The instructions 1324 may further be transmitted or received over a communications network 1326 using a transmission medium. The instructions 1324 may be transmitted using the network interface device 1320 and any one of a number of well-known transfer protocols (e.g., HTTP). Examples of communication networks include a LAN, a WAN, the Internet, mobile telephone networks, Plain Old Telephone (POTS) networks, and wireless data networks (e.g., WiFi and WiMax networks). The term "transmission medium" shall be taken to include any intangible medium that is capable of storing, encoding or carrying instructions for execution by the machine, and includes digital or analog communications signals or other intangible media to facilitate communication of such software.

As used herein, "communicatively coupled between" means that the entities on either of the coupling must communicate through an item therebetween and that those entities cannot communicate with each other without communicating through the item.

### Additional Notes

The above detailed description includes references to the accompanying drawings, which form a part of the detailed description. The drawings show, by way of illustration but not by way of limitation, specific embodiments in which the disclosure can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a," "an," "the," and "said" are used when introducing elements of aspects of the disclosure or in the embodiments thereof, as is common in patent documents, to include one or more than one of the elements, independent of any other instances or usages of "at least one" or "one or more." In this document, the term "or" is used to refer to a nonexclusive or, such that "A or B" includes "A but not B," "B but not A," and "A and B," unless otherwise indicated.

In the appended claims, the terms "including" and "in which" are used as the plain-English equivalents of the respective terms "comprising" and "wherein." Also, in the following claims, the terms "comprising," "including," and "having" are intended to be open-ended to mean that there may be additional elements other than the listed elements, such that elements after such a term (e.g., comprising, including, having) in a claim are still deemed to fall within the scope of that claim. Moreover, in the following claims, the terms "first," "second," and "third," and so forth, are used merely as labels, and are not intended to impose numerical requirements on their objects.

Embodiments of the disclosure may be implemented with computer-executable instructions. The computer-executable instructions (e.g., software code) may be organized into one or more computer-executable components or modules. Aspects of the disclosure may be implemented with any number and organization of such components or modules. For example, aspects of the disclosure are not limited to the specific computer-executable instructions or the specific components or modules illustrated in the figures and described herein. Other embodiments of the disclosure may include different computer-executable instructions or components having more or less functionality than illustrated and described herein.

Method examples (e.g., operations and functions) described herein can be machine or computer-implemented at least in part (e.g., implemented as software code or instructions). Some examples can include a computer-readable medium or machine-readable medium encoded with instructions operable to configure an electronic device to perform methods as described in the above examples. An implementation of such methods can include software code, such as microcode, assembly language code, a higher-level language code, or the like (e.g., "source code"). Such software code can include computer readable instructions for performing various methods (e.g., "object" or "executable code"). The software code may form portions of computer program products. Software implementations of the embodiments described herein may be provided via an article of manufacture with the code or instructions stored thereon, or via a method of operating a communication interface to send data via a communication interface (e.g., wirelessly, over the internet, via satellite communications, and the like).

Further, the software code may be tangibly stored on one or more volatile or non-volatile computer-readable storage media during execution or at other times. These computer-readable storage media may include any mechanism that stores information in a form accessible by a machine (e.g., computing device, electronic system, and the like), such as, but not limited to, floppy disks, hard disks, removable magnetic disks, any form of magnetic disk storage media, CD-ROMS, magnetic-optical disks, removable optical disks (e.g., compact disks and digital video disks), flash memory devices, magnetic cassettes, memory cards or sticks (e.g., secure digital cards), RAMs (e.g., CMOS RAM and the like), recordable/non-recordable media (e.g., ROMs), EPROMS, EEPROMS, or any type of media suitable for storing electronic instructions, and the like. Such computer-readable storage medium may be coupled to a computer system bus to be accessible by the processor and other parts of the OIS.

In an embodiment, the computer-readable storage medium may have encoded a data structure for a treatment planning, wherein the treatment plan may be adaptive. The data structure for the computer-readable storage medium may be at least one of a Digital Imaging and Communications in Medicine (DICOM) format, an extended DICOM format, an XML format, and the like. DICOM is an international communications standard that defines the format used to transfer medical image-related data between various types of medical equipment. DICOM RT refers to the communication standards that are specific to radiation therapy.

In various embodiments of the disclosure, the method of creating a component or module can be implemented in software, hardware, or a combination thereof. The methods provided by various embodiments of the present disclosure, for example, can be implemented in software by using standard programming languages such as, for example, Compute Unified Device Architecture (CUDA), C, C++, Java, Python, and the like; and using standard machine learning/deep learning library (or API), such as tensorflow, torch and the like; and combinations thereof. As used herein, the terms "software" and "firmware" are interchangeable, and include any computer program stored in memory for execution by a computer.

A communication interface includes any mechanism that interfaces to any of a hardwired, wireless, optical, and the like, medium to communicate to another device, such as a memory bus interface, a processor bus interface, an Internet connection, a disk controller, and the like. The communication interface can be configured by providing configuration parameters and/or sending signals to prepare the communication interface to provide a data signal describing the software content. The communication interface can be accessed via one or more commands or signals sent to the communication interface.

The present disclosure also relates to a system for performing the operations herein. This system may be specially constructed for the required purposes, or it may comprise a general-purpose computer selectively activated or reconfigured by a computer program stored in the computer. The order of execution or performance of the operations in embodiments of the disclosure illustrated and described herein is not essential, unless otherwise specified. That is, the operations may be performed in any order, unless otherwise specified, and embodiments of the disclosure may include additional or fewer operations than those disclosed herein. For example, it is contemplated that executing or performing a particular operation before, contemporaneously with, or after another operation is within the scope of aspects of the disclosure.

In view of the above, it will be seen that the several objects of the disclosure are achieved, and other advantageous results attained. Having described aspects of the disclosure in detail, it will be apparent that modifications and variations are possible without departing from the scope of aspects of the disclosure as defined in the appended claims. As various changes could be made in the above constructions, products, and methods without departing from the scope of aspects of the disclosure, it is intended that all matter contained in the above description and shown in the accompanying drawings shall be interpreted as illustrative and not in a limiting sense.

The above description is intended to be illustrative, and not restrictive. For example, the above-described examples (or one or more aspects thereof) may be used in combination with each other. In addition, many modifications may be made to adapt a particular situation or material to the teachings of the disclosure without departing from its scope. While the dimensions, types of materials and coatings described herein are intended to define the parameters of the disclosure, they are by no means limiting and are example embodiments. Many other embodiments will be apparent to those of skill in the art upon reviewing the above description. The scope of the invention is determined with by the appended claims.

Also, in the above Detailed Description, various features may be grouped together to streamline the disclosure. This should not be interpreted as intending that an unclaimed disclosed feature is essential to any claim.

## Claims

1. A computer-implemented method (1200) comprising:
receiving (1210) multi-parametric input data representing data associated with a patient;
receiving (1220) an indication of a disease associated with the patient;
applying (1230) a machine learning technique comprising a deep convolutional neural network (DCNN) to the multi-parametric input data to generate one or more metrics corresponding to a plurality of different modalities for treating the disease associated with the patient, the DCNN being trained based on training data to establish a relationship between a plurality of characteristics of pre-treatment planning associated with known patients associated with the disease, the modality of the plurality of different modalities used to treat the disease for each of the known patients, and a treatment result of each of the known patients;
selecting (1240), based on the one or more metrics, a given modality from the plurality of different modalities to treat the disease associated with the patient; and
configuring (1250) parameters of the given modality based on a portion of the multi-parametric input data, the configuring comprising:
obtaining a template for the given modality;
populating the template based on the portion of the multi-parametric input data;
transmitting the populated template for performing radiotherapy to a radiotherapy planning system; and
automatically generating a radiotherapy treatment plan based on the populated template.

2. The method of claim 1, wherein the multi-parametric input data comprise one or more image features including imaging features, electronic medical record (EMR) information, outcome metrics, and a size, volume, or intensity of a region of interest.

3. The method of claim 2, wherein the multi-parametric input data further comprises one or more features including radiomics imaging features including texture and gradients, and the outcome metrics comprise toxicities, disease-free survival information for previous patients with the disease, or reimbursement information for the plurality of different treatment modalities.

4. The method of claim 3, wherein the one or more metrics include quality assurance (QA) information for each of the plurality of different modalities, wherein the QA information identifies a particular value associated with a recommended course of radiation therapy treatment.

5. The method of claim 1, wherein a first of the plurality of different modalities to treat the disease comprises a type of radiotherapy, including external beam radiation therapy, gamma knife therapy, stereotactic radiation therapy, or proton therapy.

6. The method of claim 1, wherein the training data comprises different sets of government or professional body regulations for treating the disease, each set of government or professional body regulations being associated with a respective geographic region of a plurality of geographic regions, and wherein the training data comprises reimbursement information of each of the plurality of different modalities for each of the plurality of geographic regions.

7. The method of claim 1, wherein the operations further comprise:
computing a quality score associated with a medical facility based on the given modality selected to treat the disease associated with the patient and a treatment result associated with treating the patient with the given modality; and
causing the medical facility to be audited for treatment improvement in response to determining that the quality score is in a range between first and second values.

8. The method of claim 1, wherein the operations further comprise training the machine learning technique by performing a series of training steps comprising:
receiving a portion of training data corresponding to a given known patient of the known patients, the portion of the training data comprising characteristics of pre-treatment planning associated with the given known patient, the modality used to treat the disease for the given known patient, and the treatment result of the given known patient;
applying the machine learning technique to the characteristics of pre-treatment planning associated with the given known patient to estimate a set of modalities for treating the disease of the given known patient;
comparing the modality used to treat the disease for the given known patient with the estimated set of modalities for treating the disease of the given known patient;
computing a loss function based on a deviation parameter and a treatment result parameter, wherein the deviation parameter is determined based on a result of comparing the modality used to treat the disease for the given known patient with the estimated set of modalities, and wherein the treatment result parameter is determined based on the treatment result of the given known patient; and
updating one or more parameters of the machine learning technique based on the computed loss.

9. The method of claim 8, wherein the machine learning technique is configured to generate a weight for each of the set of modalities for treating the disease of the given known patient, the weight being generated based on the computed loss function.

10. The method of claim 8, wherein the operations further comprise:
training the machine learning technique to generate a set of modalities for treating a second disease based on additional training data comprising a plurality of characteristics of pre-treatment planning associated with known patients associated with the second disease, a modality of a plurality of different modalities used to treat the second disease for each of the known patients, and a treatment result of each of the known patients associated with the second disease.

11. The method of claim 1, wherein the operations further comprise:
ranking the plurality of different modalities for treating the disease associated with the patient based on the one or more metrics;
determining that the given modality selected to treat the disease associated with the patient is associated with a lower rank than a second modality of the plurality of different modalities; and
comparing a treatment result associated with treating the patient with the given modality with an estimated result of the second modality.

12. The method of claim 11, wherein the operations further comprise generating a prompt in response to determining that the treatment result associated with treating the patient with the given modality has a lower score than a score associated with the estimated result of the second modality.

13. The method of claim 12, wherein the operations further comprise generating an audit based on the prompt to flag a physician and a protocol used by the physician to treat the disease.

14. A system comprising:
a memory; and
one or more processors that, when executing instructions stored in the memory, are configured to perform the method of any preceding claim.

15. A computer readable medium encoded with instructions operable to configure an electronic device to perform the method of any preceding claim.

## Patentansprüche

1. Computerimplementiertes Verfahren (1200), umfassend:
Empfangen (1210) von multiparametrischen Eingabedaten, die Daten repräsentieren, die einem Patienten zugeordnet sind;
Erhalten (1220) einer Indikation einer Krankheit, die dem Patienten zugeordnet ist;
Anwenden (1230) einer Maschinenlerntechnik, die ein tiefes neuronales Faltungsnetz (DCNN) umfasst, auf die multiparametrischen Eingabedaten, um eine oder mehrere Metriken zu erzeugen, die mehreren verschiedenen Modalitäten zum Behandeln der Krankheit, die dem Patienten zugeordnet ist, entsprechen, wobei das DCNN basierend auf Trainingsdaten trainiert wird, um eine Beziehung zwischen mehreren Charakteristiken einer Vorbehandlungsplanung, die bekannten der Krankheit zugeordneten Patienten zugeordnet ist, der Modalität von den mehreren verschiedenen Modalitäten, die zum Behandeln der Krankheit für jeden der bekannten Patienten verwendet werden, und einem Behandlungsergebnis jedes der bekannten Patienten herzustellen;
Auswählen (1240) einer gegebenen Modalität aus den mehreren verschiedenen Modalitäten basierend auf der einen oder den mehreren Metriken, um die Krankheit, die dem Patienten zugeordnet ist, zu behandeln; und
Konfigurieren (1250) von Parametern der gegebenen Modalität basierend auf einem Teil der multiparametrischen Eingabedaten, wobei das Konfigurieren Folgendes umfasst:
Erhalten einer Vorlage für die gegebene Modalität;
Füllen der Vorlage basierend auf dem Teil der multiparametrischen Eingabedaten;
Senden der aufgefüllten Vorlage zum Durchführen von Strahlentherapie an ein Strahlentherapieplanungssystem; und
automatisches Erzeugen eines Strahlentherapiebehandlungsplans basierend auf der ausgefüllten Vorlage.

2. Verfahren nach Anspruch 1, wobei die multiparametrischen Eingabedaten ein oder mehrere Bildmerkmale umfassen, die Bildgebungsmerkmale, Informationen von elektronischen Krankenakten (EMR-Informationen), Ergebnismetriken und eine Größe, ein Volumen oder eine Intensität einer Region von Interesse umfassen.

3. Verfahren nach Anspruch 2, wobei die multiparametrischen Eingabedaten ferner ein oder mehrere Merkmale, die Radiomik-Bildgebungsmerkmale einschließlich Textur und Gradienten umfassen, umfassen und die Ergebnismetriken Toxizitäten, Informationen zu krankheitsfreiem Überleben für frühere Patienten mit der Krankheit oder Erstattungsinformationen für die mehreren verschiedenen Behandlungsmodalitäten umfassen.

4. Verfahren nach Anspruch 3, wobei die eine oder die mehreren Metriken Qualitätssicherungsinformationen (QA-Informationen) für jede der mehreren verschiedenen Modalitäten umfassen, wobei die QA-Informationen einen bestimmten Wert identifizieren, der einem empfohlenen Verlauf einer Strahlentherapiebehandlung zugeordnet ist.

5. Verfahren nach Anspruch 1, wobei eine erste der mehreren verschiedenen Modalitäten zum Behandeln der Krankheit eine Art von Strahlentherapie umfasst, die Strahlentherapie mit externem Strahl, Gammamessertherapie, stereotaktische Strahlentherapie oder Protonentherapie umfasst.

6. Verfahren nach Anspruch 1, wobei die Trainingsdaten unterschiedliche Sätze von Behörden- oder Fachverbandsverordnungen zum Behandeln der Krankheit umfassen, wobei jeder Satz von Behörden- oder Fachverbandsverordnungen einer jeweiligen geografischen Region von mehreren geografischen Regionen zugeordnet ist und wobei die Trainingsdaten Erstattungsinformationen jeder der mehreren verschiedenen Modalitäten für jede der mehreren geografischen Regionen umfassen.

7. Verfahren nach Anspruch 1, wobei die Operationen ferner Folgendes umfassen:
Berechnen einer Qualitätsbewertung, die einer medizinischen Einrichtung zugeordnet ist, basierend auf der gegebenen Modalität, die ausgewählt ist, um die Krankheit, die dem Patienten zugeordnet ist, zu behandeln, und einem Behandlungsergebnis, das der Behandlung des Patienten mit der gegebenen Modalität zugeordnet ist; und
Veranlassen, dass die medizinische Einrichtung als Reaktion auf das Bestimmen, dass die Qualitätsbewertung in einem Bereich zwischen einem ersten und einem zweiten Wert liegt, auf eine Behandlungsverbesserung überprüft wird.

8. Verfahren nach Anspruch 1, wobei die Operationen ferner ein Trainieren der Maschinenlerntechnik durch Durchführen einer Reihe von Trainingsschritten umfassen, die Folgendes umfassen:
Empfangen eines Teils von Trainingsdaten, die einem gegebenen bekannten Patienten von den bekannten Patienten entsprechen, wobei der Teil der Trainingsdaten Charakteristiken einer Vorbehandlungsplanung, die dem gegebenen bekannten Patienten zugeordnet ist, die Modalität, die zum Behandeln der Krankheit für den gegebenen bekannten Patienten verwendet wird, und das Behandlungsergebnis des gegebenen bekannten Patienten umfasst;
Anwenden der Maschinenlerntechnik auf die Charakteristiken einer Vorbehandlungsplanung, die dem gegebenen bekannten Patienten zugeordnet ist, um einen Satz von Modalitäten zum Behandeln der Krankheit des gegebenen bekannten Patienten zu schätzen;
Vergleichen der Modalität, die zum Behandeln der Krankheit für den gegebenen bekannten Patienten verwendet wird, mit der geschätzten Gruppe von Modalitäten zum Behandeln der Krankheit des gegebenen bekannten Patienten;
Berechnen einer Verlustfunktion basierend auf einem Abweichungsparameter und einem Behandlungsergebnisparameter, wobei der Abweichungsparameter basierend auf einem Ergebnis des Vergleichens der Modalität, die zum Behandeln der Krankheit für den gegebenen bekannten Patienten verwendet wird, mit dem geschätzten Satz von Modalitäten bestimmt wird und wobei der Behandlungsergebnisparameter basierend auf dem Behandlungsergebnis des gegebenen bekannten Patienten bestimmt wird; und
Aktualisieren eines oder mehrerer Parameter der Maschinenlerntechnik basierend auf dem berechneten Verlust.

9. Verfahren nach Anspruch 8, wobei die Maschinenlerntechnik dazu ausgelegt ist, eine Gewichtung für jede des Satzes von Modalitäten zum Behandeln der Krankheit des gegebenen bekannten Patienten zu erzeugen, wobei die Gewichtung basierend auf der berechneten Verlustfunktion erzeugt wird.

10. Verfahren nach Anspruch 8, wobei die Operationen ferner Folgendes umfassen:
Trainieren der Maschinenlerntechnik zum Erzeugen eines Satzes von Modalitäten zum Behandeln einer zweiten Krankheit basierend auf zusätzlichen Trainingsdaten, die mehrere Charakteristiken einer Vorbehandlungsplanung, die bekannten Patienten zugeordnet ist, die der zweiten Krankheit zugeordnet sind, eine Modalität von mehreren verschiedenen Modalitäten, die zum Behandeln der zweiten Krankheit für jeden der bekannten Patienten verwendet werden, und ein Behandlungsergebnis jedes der bekannten Patienten, die der zweiten Krankheit zugeordnet sind, umfassen.

11. Verfahren nach Anspruch 1, wobei die Operationen ferner Folgendes umfassen:
Bilden einer Rangfolge der mehreren verschiedenen Modalitäten zum Behandeln der Krankheit, die dem Patienten zugeordnet ist, basierend auf der einen oder den mehreren Metriken;
Bestimmen, dass die gegebene Modalität, die zum Behandeln der dem Patienten zugeordneten Krankheit ausgewählt ist, einem niedrigeren Rang zugeordnet ist als eine zweite Modalität der mehreren verschiedenen Modalitäten; und
Vergleichen eines Behandlungsergebnisses, das der Behandlung des Patienten mit der gegebenen Modalität zugeordnet ist, mit einem geschätzten Ergebnis der zweiten Modalität.

12. Verfahren nach Anspruch 11, wobei die Operationen ferner ein Erzeugen einer Aufforderung als Reaktion auf das Bestimmen umfassen, dass das Behandlungsergebnis, das der Behandlung des Patienten mit der gegebenen Modalität zugeordnet ist, eine niedrigere Bewertung aufweist als eine Bewertung, die dem geschätzten Ergebnis der zweiten Modalität zugeordnet ist.

13. Verfahren nach Anspruch 12, wobei die Operationen ferner ein Erzeugen einer Überprüfung basierend auf der Aufforderung umfassen, um einen Arzt und ein Protokoll, das von dem Arzt zum Behandeln der Krankheit verwendet wird, zu kennzeichnen.

14. System, umfassend:
einen Speicher; und
ein oder mehrere Prozessoren, die beim Ausführen von Anweisungen, die in dem Speicher gespeichert sind, dazu ausgelegt sind, das Verfahren nach einem der vorhergehenden Ansprüche durchzuführen.

15. Computerlesbares Medium, das mit Anweisungen codiert ist, die dazu dienen, eine elektronische Vorrichtung dazu zu konfigurieren, das Verfahren nach einem der vorhergehenden Ansprüche durchzuführen.

## Revendications

1. Procédé mis en œuvre par ordinateur (1200) comprenant :
la réception (1210) de données d'entrée multi-paramétriques représentant des données associées à un patient ;
la réception (1220) d'une indication d'une maladie associée au patient ;
l'application (1230) d'une technique d'apprentissage automatique comprenant un réseau de neurones convolutionnel profond (DCNN) aux données d'entrée multi-paramétriques pour générer une ou plusieurs métriques correspondant à une pluralité de modalités différentes pour traiter la maladie associée au patient, le DCNN étant entraîné sur la base de données d'apprentissage pour établir une relation entre une pluralité de caractéristiques de planification de prétraitement associées à des patients connus associés à la maladie, la modalité de la pluralité de modalités différentes utilisée pour traiter la maladie pour chacun des patients connus, et un résultat de traitement de chacun des patients connus ;
la sélection (1240), sur la base des un ou plusieurs métriques, d'une modalité donnée parmi la pluralité de modalités différentes pour traiter la maladie associée au patient ; et
la configuration (1250) de paramètres de la modalité donnée sur la base d'une partie des données d'entrée multi-paramétriques, la configuration comprenant :
l'obtention d'un modèle pour la modalité donnée ;
le remplissage du modèle sur la base de la partie des données d'entrée multi-paramétriques ;
la transmission du modèle rempli pour effectuer une radiothérapie à un système de planification de radiothérapie ; et
la génération automatique d'un plan de traitement de radiothérapie sur la base du modèle rempli.

2. Procédé selon la revendication 1, les données d'entrée multi-paramétriques comprenant une ou plusieurs caractéristiques d'image comportant des caractéristiques d'imagerie, des informations de dossier médical électronique (EMR), des métriques de résultat, et une taille, un volume ou une intensité d'une région d'intérêt.

3. Procédé selon la revendication 2, les données d'entrée multi-paramétriques comprenant en outre une ou plusieurs caractéristiques comportant des caractéristiques d'imagerie radiomique comportant une texture et des gradients, et les métriques de résultat comprenant des toxicités, des informations de survie sans maladie pour des patients précédents atteints de la maladie, ou des informations de remboursement pour la pluralité de modalités de traitement différentes.

4. Procédé selon la revendication 3, les une ou plusieurs métriques comportant des informations d'assurance qualité (QA) pour chacune de la pluralité de modalités différentes, les informations QA identifiant une valeur particulière associée à une série de traitement recommandée par radiothérapie.

5. Procédé selon la revendication 1, une première de la pluralité de modalités différentes pour traiter la maladie comprenant un type de radiothérapie, incluant la radiothérapie par faisceau externe, la radiothérapie par gamma-couteau, la radiothérapie stéréotaxique ou la protonthérapie.

6. Procédé selon la revendication 1, les données d'apprentissage comprenant différents ensembles de régulations gouvernementales ou professionnelles pour traiter la maladie, chaque ensemble de règlementations gouvernementales ou professionnelles étant associé à une région géographique respective d'une pluralité de régions géographiques, et les données d'apprentissage comprenant des informations de remboursement de chacune de la pluralité de modalités différentes pour chacune de la pluralité de régions géographiques.

7. Procédé de la revendication 1, les opérations comprenant en outre :
le calcul d'un score de qualité associé à une installation médicale sur la base de la modalité donnée sélectionnée pour traiter la maladie associée au patient et d'un résultat de traitement associé au traitement du patient avec la modalité donnée ; et
le fait de faire auditer l'installation médicale pour améliorer le traitement en réponse à la détermination que le score de qualité se situe dans une plage comprise entre des première et deuxième valeurs.

8. Procédé selon la revendication 1, les opérations comprenant en outre l'entraînement de la technique d'apprentissage automatique en effectuant une série d'étapes d'entraînement comprenant :
la réception d'une partie des données d'apprentissage correspondant à un patient connu donné des patients connus, la partie des données d'apprentissage comprenant des caractéristiques de planification de prétraitement associées au patient connu donné, la modalité utilisée pour traiter la maladie pour le patient connu donné, et le résultat de traitement du patient connu donné ;
l'application de la technique d'apprentissage automatique aux caractéristiques de planification de prétraitement associées au patient connu donné afin d'estimer un ensemble de modalités de traitement de la maladie du patient connu donné ;
la comparaison de la modalité utilisée pour traiter la maladie du patient connu donné avec l'ensemble estimé de modalités de traitement de la maladie du patient connu donné ;
le calcul d'une fonction de perte sur la base d'un paramètre de déviation et d'un paramètre de résultat de traitement, le paramètre de déviation étant déterminé sur la base d'un résultat de comparaison de la modalité utilisée pour traiter la maladie pour le patient connu donné avec l'ensemble estimé de modalités, et le paramètre de résultat de traitement étant déterminé sur la base du résultat de traitement du patient connu donné ; et
la mise à jour d'un ou plusieurs paramètres de la technique d'apprentissage automatique sur la base de la perte calculée.

9. Procédé selon la revendication 8, la technique d'apprentissage automatique étant configurée pour générer un poids pour chacun de l'ensemble de modalités pour traiter la maladie du patient connu donné, le poids étant généré sur la base de la fonction de perte calculée.

10. Procédé de la revendication 8, les opérations comprenant en outre :
l'entraînement de la technique d'apprentissage automatique pour générer un ensemble de modalités de traitement d'une deuxième maladie sur la base de données d'apprentissage supplémentaires comprenant une pluralité de caractéristiques de planification de prétraitement associées à des patients connus associés à la deuxième maladie, d'une modalité d'une pluralité de modalités différentes utilisées pour traiter la deuxième maladie pour chacun des patients connus, et d'un résultat de traitement de chacun des patients connus associés à la deuxième maladie.

11. Procédé de la revendication 1, les opérations comprenant en outre :
le classement de la pluralité de modalités différentes pour traiter la maladie associée au patient sur la base des une ou plusieurs métriques ;
la détermination du fait que la modalité donnée sélectionnée pour traiter la maladie associée au patient est associée à un rang inférieur à une deuxième modalité de la pluralité de modalités différentes ; et
la comparaison d'un résultat de traitement associé au traitement du patient avec la modalité donnée avec un résultat estimé de la deuxième modalité.

12. Procédé selon la revendication 11, les opérations comprenant en outre la génération d'une invite en réponse à la détermination que le résultat de traitement associé au traitement du patient avec la modalité donnée a un score inférieur à un score associé au résultat estimé de la deuxième modalité.

13. Procédé selon la revendication 12, les opérations comprenant en outre la génération d'un audit sur la base de l'invite pour signaler un médecin et un protocole utilisé par le médecin pour traiter la maladie.

14. Système comprenant :
une mémoire ; et
un ou plusieurs processeurs qui, lors de l'exécution d'instructions stockées dans la mémoire, sont configurés pour exécuter le procédé de l'une quelconque des revendications précédentes.

15. Support lisible par ordinateur codé avec des instructions utilisables pour configurer un dispositif électronique pour réaliser le procédé selon l'une quelconque des revendications précédentes.
